# EUROPÄISCHE PATENTSCHRIFT

(11) **EP 1 749 021 B1**
(45) Veröffentlichungstag und Bekanntmachung des Hinweises auf die Patenterteilung: **03.06.2009**
(21) Anmeldenummer: 05739905.7
(22) Anmeldetag: 21.04.2005
(51) Int. Cl.: C07K 1/22

(54) **VERFAHREN UND KIT ZUR ISOLIERUNG PHOSPHORYLIERTER PEPTIDE**
METHOD AND KIT FOR ISOLATING PHOSPHORYLATED PEPTIDES
PROCEDE ET NECESSAIRE POUR ISOLER DES PEPTIDES PHOSPHORYLES

(30) Priorität: 14.05.2004 EP 04011468
(43) Veröffentlichungstag der Anmeldung: 07.02.2007
(73) Patentinhaber: Merck Patent GmbH, 64293 Darmstadt (DE)
(72) Erfinder: ANDERS, Jonas, 64293 Darmstadt (DE); SCHWAEMMLE, Achim, 64283 Darmstadt (DE); ANDRECHT, Sven, 64347 Griesheim (DE); HENDRIKS, Robertus, 69121 Heidelberg (DE); BOERNER, Anette, 64291 Darmstadt (DE); RUETER, Cora, 12589 Berlin (DE)
(86) Internationale Anmeldenummer: PCT/EP2005/004272
(87) Internationale Veröffentlichungsnummer: WO 2005/111062

(56) Entgegenhaltungen:
- D.E. KALUME ET AL: "Tackling the phosphoproteome: tools and strategies" CURRENT OPIONION IN CHEMICAL BIOLOGY, Bd. 7, 2003, Seiten 64-69, XP002349472 in der Anmeldung erwähnt
- VLADKA GABERC-POREKAR, ET AL: "Perspectives of immonilized-metal affinity chromatography" JOURNAL OF BIOCHEMICAL AND BIOPHYSICAL METHODS, Bd. 49, 2001, Seiten 335-360, XP002349473

## Beschreibung

Die Erfindung betrifft ein Verfahren und einen Kit zur Isolierung von phosphorylierten Peptiden aus komplexen Mischungen unter Verwendung von Trägermaterialien mit Chelatliganden auf Silica-Basis und alkalischen Elutionspuffern. In bevorzugten Ausführungsformen ermöglicht das erfindungsgemäße Verfahren die Isolierung der Phosphopeptide aus komplexen Probelösungen mit reproduzierbar hoher Ausbeute und gleichzeitig mit hoher Reinheit und es erlaubt eine effiziente Ionisierung und Detektion der isolierten Phosphopeptide sowohl durch MALDI-TOF- als auch durch ESI-Massenspektrometrie ohne zusätzlich notwendige Verfahrensschritte wie etwa einer chromatographischen Entsalzung der Probe.

Mit der Sequenzierung des menschlichen Genoms erhält die Wissenschaft Zugang zum individuellen genetischen Code eines jeden Menschen. Daraus ergeben sich Informationen über seine Abstammung und Herkunft. Für die Untersuchung der biologischen Funktion einzelner Gene bzw. der entsprechenden Proteine ist diese Information aber nicht ausreichend. Das komplexe Netzwerk einer Zelle lässt sich nicht einfach durch das Entschlüsseln der genomischen DNA eines Menschen charakterisieren. Eine Untersuchung der Proteine, die vom Genom kodiert werden, muss sich der Genomanalyse anschließen, da nur mit dieser Zusatzinformation das dynamische Geschehen des menschlichen Organismus auf molekularer Ebene beschreibbar ist. Auch gibt es oftmals nur eine geringe Korrelation zwischen der Gen-Transkription und dem entsprechenden Translationsprodukt, so dass nur mit Hilfe der Proteomanalyse erkennbar wird, welche Proteine unter gegebenen Einflüssen wie stark exprimiert und eventuell posttranslational verändert werden. Die quantitative Analyse der Expression eines Proteins und die Untersuchung eventueller posttranslationaler Modifikationen sind jedoch Grundvoraussetzung für das Verständnis der Funktion eines bestimmten Proteins.

Die Komplexität des zellulären Proteoms steigt exponentiell an, wenn die möglichen posttranslationalen Modifikationen der Proteine berücksichtigt werden. Die dynamische posttranslationale Modifikation von Proteinen ist oftmals entscheidend für den Erhalt und die Regulation der Proteinstruktur und -funktion. Zur Zeit sind mehrere hundert verschiedene posttranslationale Modifikationen von Proteinen bekannt, von denen die Phosphorylierung die weitaus prominenteste darstellt. Enzymatisch katalysierte Phosphorylierung und Dephosphorylierung ist ein wichtiges regulatorisches Element für die lebende Zelle. Organismen nutzen reversible Proteinphosphorylierung zur Kontrolle von so fundamentalen zellulären Prozessen wie Signaltransduktion, Zellzyklus, der Organisation des Cytoskeletts, dem Stoffwechsel sowie dem programmierten Zelltod und der Genexpression. Die transiente und reversible Phosphorylierung bestimmter Aminosäuren von entsprechenden an diesen Prozessen beteiligten Proteinen dient dabei der stringenten Kontrolle von Aktivität, Stabilität, Lokalisierung oder Interaktionen. Eine umfassende Analyse von Phosphoproteinen und die Bestimmung von Phosphorylierungsstellen ist demnach Voraussetzung für das Verständnis komplexer biologischer Systeme und der molekularen Grundlagen für die Entstehung von Krankheiten.

Gerade die an regulatorischen Prozessen beteiligten Proteine sind in der Zelle jedoch in der Regel nur in relativ niedriger Abundanz vertreten. Zudem ist die transiente Phosphorylierung von Proteinen selten stöchiometrisch, so dass die phosphorylierte Spezies in der Regel gemeinsam mit der unphosphorylierten Form vorliegt. Die Analyse und Identifizierung von Phosphoproteinen und die Identifizierung von Phosphorylierungsstellen muss zudem in der Regel aufgrund der geringen zugänglichen Mengen durch empfindliche, massenspektrometrische Methoden erfolgen. Diese Methoden erfordern typischerweise die enzymatische Spaltung des zu analysierenden Phosphoproteins in Fragmente, meistens in tryptische Peptide. Phosphorylierte Aminosäuren treten jedoch nur in bestimmten Peptiden auf, welche Erkennungssequenzen für die an der Phosphorylierung beteiligten Enzyme enthalten. Nebst den vorher genannten stöchiometrischen Effekten liegen Phosphopeptide deshalb selbst im Falle der Analyse eines zur Homogenität gereinigten Phosphoproteins im Gemisch mit nicht phosphorylierten Peptiden desselben Proteins vor. Bei einer Analyse können Peptide unterhalb einer bestimmten relativen Abundanz im Peptidgemisch nicht mehr sicher detektiert werden. Zum Einen können schwache kleine Signale im Hintergrundrauschen verschwinden und zum Zweiten kompetitieren hoch- abundante Peptide um die Ionisierungs-Energie, so dass niedrig abundante Peptide ohne vorherige Anreicherung möglicherweise gar nicht ionisiert und damit auch nicht detektiert werden können. Es wird abgeschätzt, daß im menschlichen Proteom etwa 100.000 potentielle Phosphorylierungsstellen in der Primärsequenz entsprechender Proteine codiert sind, von denen jedoch bisher nur etwa 2000 identifiziert werden konnten. Strategien zur selektiven und effizienten Anreicherung phosphorylierter Peptide aus proteolytischen Extrakten phosphorylierter Proteine mit hoher Ausbeute sind demnach integraler Bestandteil einer umfassenden Analyse des Phosphoproteoms.

Eine reproduzierbare Anreicherungsmethode für phosphorylierte Peptide zur Analyse des Phosphoproteoms muss hierbei wegen der oft geringen Abundanz der Phosphoproteine und des substöchiometrischen Auftretens der Phosphorylierung der entsprechenden Aminosäuren möglichst quantitative Ausbeute des entsprechenden Phosphopeptides liefern, um auch niedrig abundante Phosphopeptide einer Analyse zugänglich zu machen. Gleichzeitig muss die Anreicherungsmethode quantitative Reinheit liefern, um trotz der oben aufgeführten stöchiometrischen Effekte eine direkte Analyse der Phosphopeptide in der Probe zu erlauben.

Schätzungsweise ein Drittel aller in einer typischen Säugerzelle enthaltenen Proteine kann potentiell durch Phosphorylierung posttranslational modifiziert werden. Die dafür verantwortlichen Enzyme, die Kinasen, repräsentieren etwa 1 - 3 % des exprimierten Genoms einer typischen Säugerzelle. Die Modifikation des Proteins durch Phosphorylierung kann dabei an den Seitenketten der Aminosäuren Serin, Threonin, Tyrosin, Histidine, Arginine, Lysine, Cysteine, Glutamat und Aspartat erfolgen. Jedoch sind die drei Aminosäuren Serin (etwa 90 %), Threonin (etwa 10 %) und Tyrosin (etwa 0.05 %) die bevorzugten Reste. Verfahren zur Anreicherung phosphorylierter Peptide sollte demnach auf die phosphorylierten Derivate mindestens der drei Aminosäuren Serin, Threonin und Tyrosin im gleichen Maß anwendbar sein.

Traditionelle Methoden zur Analyse von Phosphoryllerungstellen in Proteinen benutzen die Möglichkeit, radioaktive Phosphorisotope ([P³²], [P³³]) zur Markierung von Phosphoproteinen einzusetzen, um die Probe anschließend nach dem Stand der Technik durch Gelelektrophorese, enzymatischen Verdau und Sequenzierung, respektive Peptid-Mapping zu analysieren. Um quantitative Unterschiede innerhalb des Phosphoproteoms zweier unterschiedlicher Zellzustände zu bestimmen, wird die Intensität der radioaktiven Strahlung zweier Proben miteinander verglichen. Die Nachteile eines solchen Verfahrens sind zum einen der Einsatz von radioaktiver Strahlung an sich und die dadurch bedingte Kontamination von z.B. Mess-Geräten. Zudem sind für diese Methode lediglich stoffwechselaktive Proben einsetzbar. Nicht stoffwechselaktive, klinisch relevante Proben, wie etwa Gewebeschnitte von Krebspatienten, sind mit diesem Verfahren nicht zugänglich. Phosphoproteine haben zudem unterschiedliche Umsatzraten für die enzymkatalysierte Phosphorylierung, wodurch quantitative Aussagen hinsichtlich des Zusammenhangs der Abundanz des Proteins und der Einbaurate von [P³²] bzw. [P³³] verfälscht werden können. Eine quantitative Ausbeute und Reinheit im Sinne der vorliegenden Erfindung kann somit durch Markierung von Phosphoproteinen mit radioaktiven Phosphorisotopen nach dem Stand der Technik nicht erreicht werden.

Eine weitere Methode zur Anreicherung von Phosphoproteinen ist die Affinitätsanreicherung mittels phosphospezifischer Antikörper. Dazu werden Antikörper eingesetzt, die spezifisch an Phosphoamino- Epitope von Phosphoserin, -threonin oder -tyrosin binden. Jedoch sind bis jetzt nicht für alle Epitope von Phospho-Aminosäuren spezifische Antikörper gefunden worden. Antikörper, im Besonderen die gegen Phosphoserin und -threonin gerichteten, können oftmals aufgrund von sterischen Hindernissen nicht mit den phosphorylierten Aminosäuren reagieren. Falls bestimmte Phosphoproteine durch die eingesetzten Antikörper nur mit geringer Affinität gebunden werden, kann der hohe Anteil unspezifischer Bindung von anderen, nicht phosphorylierten Proteinen eine Analyse der Phosphorylierungsstellen des Proteins verhindern.

Die Immunaffinitätsanreicherung von Phosphoproteinen oder -peptiden nach dem Stand der Technik unterliegt damit mehreren Limitationen. Eine quantitative Ausbeute und Reinheit kann mit dieser Methode somit nicht erreicht werden.

Eine indirekte Methode zur Anreicherung von Proteinen/Peptiden mit Phosphoserin und -threonin ist die chemische Umwandlung der Phosphatgruppen des Proteins oder Peptids. Dabei werden die Gruppen chemisch modifiziert und mit Affinitäts-Tags, z.B. Biotin, versehen oder zur Anreicherung kovalent immoblisisiert. Nachteil dieser Methoden ist das Auftreten von Nebenreaktionen während der chemischen Modifizierung, so dass beispielsweise eine unerwünschte Modifikation von Aminosäuren oder eine unspezifische Fragmentierung von Proteinen und Peptiden auftritt, die die Identifizierung der Peptide im Massenspektrum erschwert. Dazu kommt, dass mehrstufige chemische Modifikationen aufwändig in der Durchführung sind und zumeist größere Probenmengen benötigen.

Eine weitere Methode zur Anreicherung von phosphorylierten Peptiden aus komplexen Mischungen ist die immobilisierte Metallchelat-Affinitätschromatographie (IMAC). Die Anreicherung von Phosphopeptiden durch IMAC ist einfach durchzuführen, erfordert prinzipiell keine Modifikation der Proben vor der Anreicherung, ist anwendbar auf nicht stoffwechselaktive Proben, unterscheidet nicht zwischen verschiedenen Phosphoaminosäuren und ist zudem relativ preisgünstig durchzuführen. Aus diesem Grund ist die IMAC derzeit die vorteilhafteste Methode zur Isolierung von Phosphopeptiden.

IMAC wird nach dem Stand der Technik mit Hilfe von Chromatographiematerialien durchgeführt, welche zur Bindung von Metallionen mit Chelatbildnern oberflächenmodifiziert sind [zusammengefasst in Gaberc-Porekar V. und Menart V. (2001): "Perspectives of immobilized-metal affinity chromatography" J. Biochem. Biophys. Methods, 49, 335-360]. Für die Phosphopeptid-Anreicherung sind die Oberflächenmodifikationen Iminodiacetat (IDA), ein dreizähniger Chelatbildner [Porath J. und Olin B (1983): "Immobilized metal ion affinity adsorption and immobilized metal ion affinity chromatography of biomaterials" Biochemistry 22, 1621-1630], sowie Nitrilotriacetat (NTA), ein vierzähniger Chelatbildner [Hochuli E., Doebeli H and Schacher A (1987): "New metal chelate adsorbent selectivite for proteins and peptides containing neighbouring histidine residues" J. Chrom., 411, 177-184], sowie IPAC (*Immobilized Phosphonic Acid* Chelating) [Kaffashan A. und Zeng C. (2003). "Evaluation of commercially available IMAC Kits: Millipore ZipTipMC, IPAC beads and Pierce Swell Gel Gallium Chelated disks" Poster präsentiert auf der 51. ASMS Conference on Mass Spectrometry and Allied Topics, 2003, Montreal, Canada] beschrieben. Neben ihrer Effizienz bei der Anreicherung von Phosphopeptiden muß auch die Zugänglichkeit der Liganden beachtet werden. Die meisten Liganden sind nur in mehrstufigen Reaktionen zugänglich und somit teuer und aufwändig in der Herstellung. Wünschenswert wären daher Trägermaterialien mit Liganden, die nicht nur eine effektive Anreicherung ermöglichen, sondern auch einfach und schnell und somit kostengünstig hergestellt werden können.

Neben der Auswahl eines geeigneten Trägermaterials und Liganden wird die Effizienz der IMAC, d.h. die Reinheit und die Ausbeute der isolierten Phosphopeptide, hauptsächlich durch folgende Faktoren bestimmt:
1. Auswahl der Pufferbedingungen bei der Bindung
2. Auswahl der Pufferbedingungen bei der Elution
3. Auswahl des Metallions zur Aktivierung der Chelatliganden

### Zu 1.:

Zur Bindung eines Phosphopeptides an immobilisierte Metallionen tragen alle in Polypeptiden vorhandenen Elektronendonoren bei, insbesondere die Seitenkette von Histidin, aber auch anderer basischer Aminosäuren und saurer Aminosäuren wie Glutamat und Aspartat, sowie die Phosphatgruppe an der Seitenkette phoshorylierter Aminosäuren. Somit kann erwartet werden, dass die Selektivität der IMAC für Phosphopeptide problematisch ist in Bezug auf die gewünschte Reinheit des Phosphopeptides vor der Analyse. Nach dem Stand der Technik erfolgt die Bindung von Phosphopeptiden an immobilisierte Metallionen deshalb bei saurem pH-Wert (2,5 bis 3,5), um durch die Protonierung der Seitenketten der basischen Aminosäuren eine höhere Spezifität gegenüber Phosphopeptiden zu erhalten. Die durch die Carboxylgruppe saurer Aminosäuren vermittelte Affinität zu immobilisierten Metallen wird dadurch jedoch nicht eingeschränkt. Die unspezifische Bindung von sauren Peptiden an immobilisierte Metallionen ist demnach auch das Hauptproblem der nach dem Stand der Technik durchgeführten IMAC zur Anreicherung von Phosphopeptiden [Kalume D.E., Molina H. und Pandey A. (2003). "Tackling the phosphoproteome: tools and strategies" Current Opinion in Chemical Biolog 7, 64-69]. IMAC techni en sind auch bei Vladka, Gabere-Pomekar et al: "Perspectives of immobilized-metal affinity chromatography, Journal of Biochemical and Biophysical Methods, Bd. 49, 2001, seiten 335-360 offenbart.

### Zu. 2.:

Ein weiterer Punkt bei der IMAC von Phosphopeptiden ist die quantitative Elution der gebundenen Phosphopeptide von den immobilisierten Metallionen. Zur Elution von Phosphopeptiden sind nach dem Stand der Technik verschiedene Basen wie NaOH, NH₄OH oder 0,1 M Carbonat beschrieben oder kompetitive Elution durch Phosphationen bei pH 8,4 bis 9,4 in Kombination mit einem organischen Polymer als Trägermaterial [Heintz et al., Electrophoresis 2004, 25, 1149-1159]. Eine quantitative Elution der gebundenen Phosphopeptide mit diesen Methoden ist jedoch nicht möglich.

### Zu 3.:

Nach dem Stand der Technik können verschiedene Ionen der Übergangsmetalle und dreiwertige Metallionen der dritten Hauptgruppe zur Anreicherung phosphorylierter Peptide durch IMAC eingesetzt werden, die hinsichtlich ihrer Effizienz und Selektivität je nach den sonstigen Isolierungsbedingungen (z.B. Ligand, Bindungs- und Elutionsbediungen) schwanken.

Als geeignete Ionen werden beispielsweise Gallium (III), Eisen (III), Aluminium (III) und Zirkonium (IV) genannt.

Bisher konnte jedoch ungeachtet intensiver Forschungsarbeit keine Methode entwickelt werden, die eine quantitative Ausbeute bei gleichzeitiger quantitativer Reinheit erlaubt.

Aufgabe der vorliegenden Erfindung war es daher, ein Verfahren für die Isolierung von Phosphopeptiden zu entwickeln, das eine nahezu quantitative Isolierung bei gleichzeitig hoher Reinheit der Produkte ermöglicht.

Es wurde gefunden, dass die Verwendung von Trägermaterialien auf Basis von Silica in Kombination mit bestimmten basischen Elutionspuffer eine besonders effiziente Isolierung von Phosphopeptiden ermöglicht. Besonders hohe Effizienz zeigt sich bei dem Einsatz eines neuen Trägermaterials mit Chelatliganden auf Basis von Ethylendiamindiessigsäure.

Gegenstand der vorliegenden Erfindung ist ein Verfahren zur Anreicherung von Phosphopeptiden, gekennzeichnet durch folgende Verfahrensschritte:
a) Bereitstellung eines Trägermaterials mit Chelatliganden auf Basis von Silica
b) Aktivierung des Trägermaterials aus Schritt a) mit Übergangsmetallionen, Oxiden oder Oxidhydraten von Übergangsmetallionen oder dreiwertigen Ionen von Metallen der dritten Hauptgruppe
c) Inkontaktbringen einer phosphopeptidhaltigen Probe mit dem aktivierten Trägermaterial in Anwesenheit eines Bindungspuffers
d) Entfernen des Überstands bestehend aus dem Bindungspuffer und dem nicht gebundenen Teil der Probe
e) optional Waschen des Trägermaterials
f) Elution der Phosphopeptide mit einem Elutionspuffer, der einen pH-Wert > 10 aufweist und Alkali-, Erdalkali- oder Ammoniumsalze des Thiocyanats, der Säuren der Komplexliganden Nitrito, Isocyano, Nitril, Isocyanato, Isothiocyanato, Azido, Ethylendiamin, Isonitril, Fulminato und Cyano und/oder der Sauerstoffsäuren des Phosphors, Schwefels, Vanadiums, Rutheniums, Niobs, Tantals, Wolframs oder des Molybdäns enthält und/oder Chelatbildner wie EDTA, EGTA oder Salicylsäure.

In einer bevorzugten Ausführungsform erfolgt die Aktivierung in Schritt b) mit Eisen (III)-Ionen, besonders bevorzugt mit Zirkonium (IV)-Ionen.

In einer bevorzugten Ausführungsform erfolgt die Elution in Schritt f) mit einem Elutionspuffer, der Alkali-, Erdalkali- oder Ammoniumsalze von Sauerstoffsäuren des Phosphors oder besonders bevorzugt von Thiocyanat in einer Konzentration zwischen 0,005 und 2 mol/l enthält.

In einer weiteren bevorzugten Ausführungsform werden die in Schritt f) eluierten Phosphopeptide direkt, d.h. direkt nach einer Entsalzung oder bevorzugt direkt ohne Entsalzung, massenspektrometrisch, Dünnschicht-chromatographisch oder durch Sequenzanalyse untersucht.

In einer bevorzugten Ausführungsform wird in Schritt a) ein Trägermaterial mit Chelatliganden gemäß Formel Ia und/oder Ib bereitgestellt 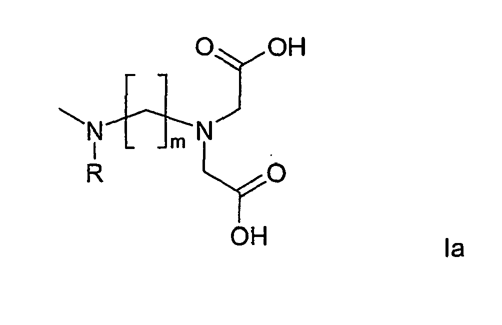 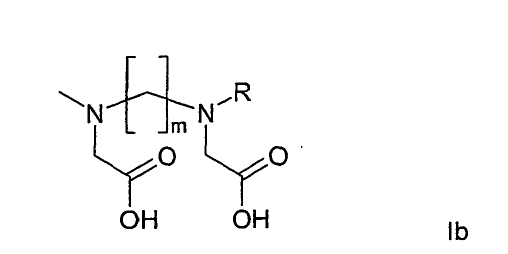 mit
R = C1 bis C6 Alkyl oder C5 bis C18 Aryl, optional ein oder mehrfach substitutiert z.B. mit Hydroxy-, C1-C4-Alkoxy-, Amino-, Alkylamino-, CN- oder Halogenresten,
m = 2 bis 8, wobei ein oder mehrere nicht benachbarte C-Atome durch O, NH, S oder -C=C- ersetzt sein können.

In einer besonders bevorzugten Ausführungsform wird in Schritt a) ein Trägermaterial mit Chelatligand bereitgestellt, das aus Magnetit-Partikeln besteht, deren Oberfläche zumindest teilweise mit Silica belegt ist.

Gegenstand der vorliegenden Erfindung ist weiterhin ein Kit zur Anreicherung von Phosphopeptiden zumindest enthaltend ein Trägermaterial mit Chelatligand auf Silica-Basis und einen Elutionspuffer, der einen pH-Wert >10 aufweist und Alkali-, Erdalkali- oder Ammoniumsalze des Thiocyanats, der Säuren der Komplexliganden Nitrito, Isocyano, Nitril, Isocyanato, Isothiocyanato, Azido, Ethylendiamin, Isonitril, Fulminato oder Cyano und/oder der Sauerstoffsäuren des Phosphors, Schwefels, Vanadiums, Rutheniums, Niobs, Tantals, Wolframs und/oder Molybdäns enthält und/oder Chelatbildner wie EDTA, EGTA oder Salicylsäure.

In einer bevorzugten Ausführungsform ist das Trägermaterial mit Chelatligand mit Eisen (III) oder bevorzugt Zirkonium (IV)-Ionen aktiviert.

In einer bevorzugten Ausführungsform enthält der Kit als Trägermaterial Magnetit-Partikel, die zumindest teilweise mit Silica überzogen sind.

In einer anderen bevorzugten Ausführungsform enthält der Kit ein Trägermaterial mit Chelatliganden entsprechend Formel Ia und/oder Ib, mit R = Methyl und m = 2.

In einer weiteren bevorzugten Ausführungsform enthält der Kit als Elutionspuffer einen Puffer, der Alkali-, Erdalkali- oder Ammoniumsalze von Sauerstoffsäuren des Phosphors oder besonders bevorzugt von Thiocyanat in einer Konzentration zwischen 0,005 und 2 mol/l enthält.

Gegenstand der vorliegenden Erfindung ist auch ein Trägermaterial, **dadurch gekennzeichnet, dass** es Chelatliganden der Formel Ia und/oder Ib enthält 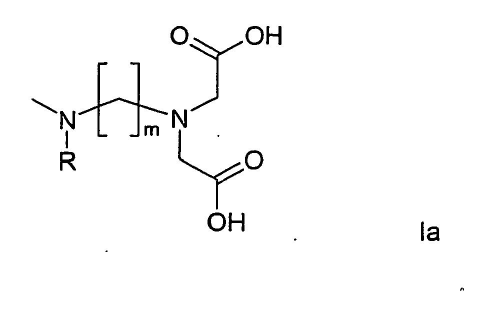 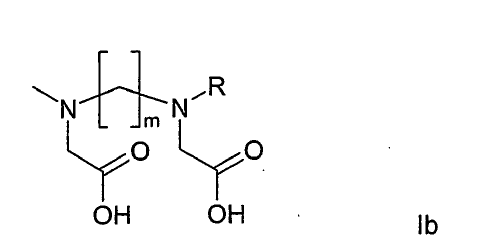 mit
R = C1 bis C6 Alkyl oder C5 bis C18 Aryl, optional ein oder mehrfach substitutiert z.B. mit Hydroxy-, C1-C4-Alkoxy-, Amino-, Alkylamino-, CN- oder Halogenresten,
m = 2 bis 8, wobei ein oder mehrere nicht benachbarte C-Atome durch O, NH, S oder -C=C- ersetzt sein können.

In einer bevorzugten Ausführungsform ist R Ethyl, besonders bevorzugt Methyl.

In einer bevorzugten Ausführungsform ist m = 2.

Abbildung 1 zeigt ein HPLC-Chromatogramm mit den Peaks der drei Modell- Peptide für die gleichzeitige qualitative und quantitative Analyse von Verfahren zur Phosphopeptid-Anreicherung durch IMAC. Nähere Angaben finden sich in Beispiel 1.

In Abbildung 2 sind Ausschnitte von ESI-LC/MS-Spektren der unprozessierten Probe und von gebundenen Peptiden nach der erfindungsgemäßen Anreicherung gezeigt. Nähere Angaben finden sich in Beispiel 6.

Abbildung 3 zeigt die Analyse von Eluaten nach der erfindungsgemäßen Anreicherung von Phosphopeptiden durch MALDI-Massenspektrometrie. Nähere Angaben finden sich in Beispiel 7.

Abbildung 4 zeigt die Analyse von Eluaten nach der erfindungsgemäßen Anreicherung von Phosphopeptiden durch Elution mit Ammoniumthiocyanat-Puffer bei alkalischem pH durch MALDI-Massenspektrometrie. Nähere Angaben finden sich in Beispiel 7.

Abbildung 5 zeigt einen Vergleich der erfindungsgemäßen Anreicherung von Phosphopeptiden mit dem Stand der Technik. Nähere Angaben finden sich in Beispiel 8.

Abbildung 6 zeigt die ESI-MS Spektren einer Anreicherung von Phosphopeptiden mit einer bevorzugten Ausführungsform des erfindungsgemäßen Verfahrens. Nähere Angaben finden sich in Beispiel 3.

Abbildung 7 zeigt exemplarisch die Synthese eines erfindungsgemäß bevorzugten Trägermaterials mit einem Chelatliganden basierend auf Ethylendiamindiacetat.

Phosphopeptide sind erfindungsgemäß Peptide, Polypeptide oder Proteine, die zumindest an einer Stelle phosphoryliert sind. Die Länge der Phosphopeptide ist unkritisch. Wichtig ist lediglich, dass die Peptide unter den für die Anreicherung eingesetzten Bedingungen (d.h. typischerweise in den Bindungspuffern) löslich sind. Im allgemeinen können daher Peptide eingesetzt werden, die bei einem pH-Wert von ca. 2,5 löslich sind. Die Löslichkeit kann dabei durch Zugabe von chaotropen Substanzen unterstützt werden. Typischerweise liegt die Länge der Peptide in den Längenbereichen wie sie nach einer enzymatischen oder chemischen Spaltung, z.B. einem tryptischen Verdau, erhalten werden. Genauso können jedoch auch kürzere oder längere Phosphopeptide mit dem erfindungsgemäßen Verfahren angereichert werden.

Eine phosphopeptidhaltige Probe ist eine Lösung, in der Phosphopeptide vorhanden sind oder zumindest vermutet werden. Zumeist ist die phosphopeptidhaltige Probe die Lösung, die nach einer chemischen oder enzymatischen Spaltung erhalten wird.

Trägermaterialien sind feste Materialien wie sie typischerweise für chromatographische oder Extraktionszwecke eingesetzt werden. Bei Trägermaterialien auf Basis von Silica handelt es sich um Materialien, die ganz aus Glas, Keramiken und/oder Silica bestehen, oder bei denen die Oberfläche zumindest teilweise mit Glas, Keramiken und/oder Silica belegt ist. Der Begriff Silica umfasst auch Materialien, bei deren Herstellung Silane verwendet wurden, die ein oder zwei organische Reste (d.h. z.B. C1 bis C8-Alkyl und/oder C5 bis C18 Aryl-Reste, insbesondere Methyl, Ethyl, n/iso-Propyl, n/tert-Butyl, Phenyl, Benzyl oder Naphthyl) tragen, d.h. sogenannte Hybrid-Materialien.

Das Trägermaterial kann beispielsweise als monolithische Säule, Platte, Partikel, Beschichtung, Faser, Filter oder andere poröse oder unporöse Struktur vorliegen. Bevorzugt liegt das Material als Partikel vor.

Bevorzugt werden erfindungsgemäß Silica-Materialien eingesetzt. Besonders bevorzugt besteht das Trägermaterial aus Magnetit-Partikeln, die zumindestens teilweise mit Silica beschichtet sind.

Zur Herstellung von Magnetit-Partikeln sind verschiedene Herstellverfahren bekannt. Beispiele sind offenbart in:
- Massart, IEEE Trans. Magn. 17, 1247-1248 (1981)
- Sugimoto, Matijevic, J. Colloid Interface Sci. 74, 227-243 (1980)
- Qu et al., J. Colloid Interface Sci. 215, 190-192 (1999)

Besonders bevorzugt erfolgt die Herstellung der Festphase aus Magnetit nach Sugimoto und Matijevic.

Vorteil magnetischer Materialien ist weiterhin ihre einfache Entfernung aus flüssigen Medien durch Anlegen eines Magnetfeldes.

Ein Beispiel für ein erfindungsgemäß besonders geeignetes Trägermaterial aus Magnetit-Partikelh deren Oberfläche mit Silica belegt ist, sind MagPrep^{®} Silica Partikel der Firma Merck KGaA, Deutschland. Ein Beispiel für erfindungsgemäß geeignete Silica-Partikel sind LiChrospher^{®} Partikel der Firma Merck KGaA, Deutschland.

Ein Trägermaterial mit Chelatligand ist ein Trägermaterial, an das Chelatliganden kovalent gebunden sind. Erfindungsgemäß geeignete Trägermaterialien mit Chelatligand sind Trägermaterialien auf Basis von Silica, an die drei-, vier- oder fünfzähnige Metall-Chelatliganden kovalent gebunden sind. Geeignete drei-, vier- oder fünfzähnige Chelatliganden sind dem Fachmann auf dem Gebiet der IMAC bekannt. Beispiele sind Iminodiacetat (IDA), ein dreizähniger Chelatbildner sowie der vierzähnige Chelatbildner Nitrilotriacetat (NTA) oder IPAC (*Immobilized Phosphonic Acid Chelating*).

Besonders bevorzugt wird erfindungsgemäß ein Trägermaterial mit einem Chelatligand basierend auf Ethylendiamindiacetat eingesetzt. Dieses Trägermaterial mit Chelatligand wird durch Umsetzung eines aktivierten Trägermaterials mit entsprechend funktionalisierten Liganden auf Basis von Ethylendiamindiactetat erzeugt.

Unter aktivierten Trägermaterialien sind erfindungsgemäß Trägermaterialien mit reaktiven Gruppen zu verstehen, die mit oder ohne Zugabe zusätzlicher Reagenzien eine kovalente Bindung mit primären und/oder sekundären Aminen eingehen können. Entsprechende aktivierte Trägermaterialien werden z.B. auch für die Einführung von Separationseffektoren in Trägermaterialien für die Chromatographie verwendet. Beispiele für aktivierte Trägermaterialien sind Materialien mit Azlacton-Gruppen oder NHS-Estem, sowie bevorzugt Trägermaterialien mit Epoxid-Gruppen. Dem Fachmann ist bekannt, welche Reaktionsbedingungen und/oder zusätzlichen Reagenzien notwendig sind, um eine kovalente Bindung des Alkylendiamins der Formel II mit einem speziellen aktivierten Träger zu erzeugen. Zur Herstellung der erfindungsgemäßen Trägermaterialien mit Chelatligand basierend auf Ethylendiamindiacetat wird ein aktiviertes Trägermaterial, bevorzugt Epoxid-aktiviertes Trägermaterial, mit den Amino-funktionalisierten Liganden der Formel II umgesetzt. 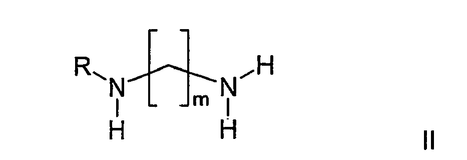
worin R = C1 bis C6 Alkyl oder C5 bis C18 Aryl, optional ein oder mehrfach substitutiert z.B. mit Hydroxy-, C1-C4-Alkoxy-, Amino-, Alkylamino-, CN- oder Halogenresten,
m = 2 bis 8, wobei ein oder mehrere nicht benachbarte C-Atome durch O, NH, S oder -C=C- ersetzt sein können.

Anschließend erfolgt die Umsetzung des modifizierten Trägermaterials mit Monohalogenessigsäure. Da ein vierzähniger Ligand erhalten werden soll, findet diese Umsetzung in der Regel mit mindestens zwei Äquivalenten Halogenessigsäure pro Ligand statt. Bevorzugt wird als Halogenessigsäure Bromessigsäure verwendet.

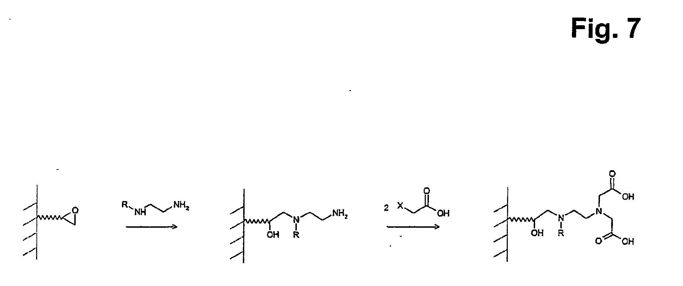

Abbildung 7 zeigt schematisch die Synthese eines erfindungsgemäßen Trägermaterials mit Chelatliganden ausgehend von einem Epoxy-aktivierten Trägermaterial. Dabei hat R die Bedeutung gemäß Formel Ia/b und X = Cl, Br oder I. Der Übersicht halber ist nur die Synthese eines Liganden gemäß Formel Ia gezeigt. Es versteht sich, dass bei der Umsetzung nicht nur - wie in dem Reaktionsschema gezeigt - ein Ligand an das Trägermaterial gebunden wird, sondern eine Vielzahl von Liganden. Die Anzahl der Liganden pro Mengeneinheit Trägermaterial hängt von der Anzahl der reaktiven Gruppen auf dem Trägermaterial ab, die für eine Anbindung der Liganden zur Verfügung stehen.

Durch die einfache und schnelle Herstellung bietet das erfindungsgemäße Trägermaterial mit Chelatligand auf Basis von Ethylendiamindiacetat neben seinen guten Eigenschaften bei der IMAC einen weiteren Vorteil, da viele andere Liganden nur mit sehr aufwändigen und langen Synthesen zugänglich sind.

Die erfindungsgemäßen Trägermaterialien mit Chelatligand auf Basis von Ethylendiamindiacetat tragen Liganden der Formel Ia und/oder Ib, da im ersten Schritt der Synthese sowohl die primäre wie auch die sekundäre Aminogruppe der Verbindung der Formel II mit dem aktivierten Trägermaterial reagieren kann. In welchem Verhältnis die beiden Aminogruppen reagieren und in welchem Verhältnis dadurch Strukturen nach Formel Ia und Ib entstehen, hängt unter anderem von dem sterischen und elektronischen Einfluß des Restes R in Formel II ab. Es ist jedoch davon auszugehen, dass in der Regel Trägermaterialien entstehen, in denen Teile der Liganden gemäß Formel Ia vorliegen und Teile der Liganden gemäß Formel Ib. 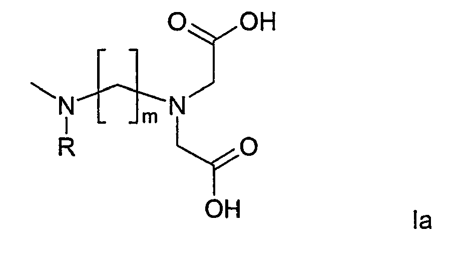 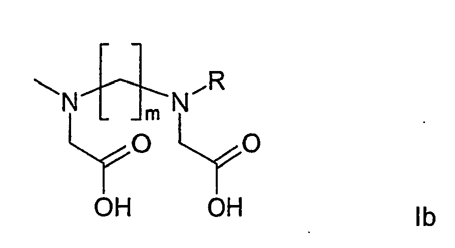 mit
R = C1 bis C6 Alkyl oder C5 bis C18 Aryl, optional ein oder mehrfach substitutiert z.B. mit Hydroxy-, C1-C4-Alkoxy-, Amino-, Alkylamino-, CN- oder Halogenresten,
m = 2 bis 8, wobei ein oder mehrere nicht benachbarte C-Atome durch O, NH, S oder -C=C- ersetzt sein können.

Besonders bevorzugt sind Trägermaterialien mit Liganden der Formel Ia und /oder Ib, in denen m = 2 ist. Weiterhin bevorzugt sind Trägermaterialien mit Liganden der Formel Ia und /oder Ib, in denen R ein kurzkettiger, möglichst kleiner Rest ist, wie R = Ethyl, Methyl, Hydroxyethyl oder Trifluormethyl. Besonders bevorzugt ist das Trägermaterial mit Liganden mit m = 2 und R = Ethyl oder Methyl, insbesondere R = Methyl. Im folgenden werden Trägermaterialien, die Chelatliganden mit m = 2 und R = Ethyl tragen Ethyl-EDDA-modifizierte Trägermaterialien genannt. Trägermaterialien, die Chelatliganden mit m = 2 und R = Methyl tragen, werden Methyl-EDDA-modifizierte Trägermaterialien genannt.

Die erfindungsgemäßen Trägermaterialien mit Chelatliganden auf Basis von Ethylendiamindiacetat eignen sich für alle Anwendungen in denen herkömmlich immobilisierte Chelatliganden eingesetzt werden. Besonders gut eignen sie sich für die Biochromatographie von Proteinen oder Peptiden, ganz besonders für die Anreicherung von Phosphopeptiden. Für andere Anwendungen als die Anreicherung von Phosphopeptiden können neben den Trägermaterialien auf Silica-Basis auch andere Trägermaterialien, wie organische Polymere oder andere anorganischen Oxide zur Herstellung der erfindungsgemäßen Trägermaterialien mit Chelatligand auf Basis von Ethylendiamindiacetat eingesetzt werden.

Die Effizienz der IMAC von Phosphopeptiden wird bestimmt durch die quantitative Ausbeute der Anreicherung und durch die quantitative Reinheit der isolierten Phosphopeptide. Wünschenswert ist eine möglichst quantitative Ausbeute der entsprechenden Phosphopeptide bei gleichzeitig quantitativer Reinheit. Als quantitative Ausbeute im Sinne der Erfindung wird hierbei eine Ausbeute an Phosphopeptid von 80%, bevorzugt von 90 % oder mehr bezeichnet. Als quantitative Reinheit im Sinne der Erfindung wird hierbei eine Reinheit an Phosphopeptid von 80%, bevorzugt von 90 % oder mehr bezeichnet.

Um die Effizienz einer Anreicherung, d.h. gleichzeitig die quantitative Ausbeute und qualitative Reinheit des Produktes zu bestimmen, wurde ein Testverfahren etabliert. Dieses Testverfahren basiert auf dem unterschiedlichen Retentionsverhalten von Peptiden bei der *reversedphase* (RP)-HPLC-Trennung und der damit verbundenen Möglichkeit zur direkten Quantifizierung der in einer Probelösung vorhandenen Peptidmenge in Relation zu einem Kalibrationsstandard durch Integration des UV-Absorptionssignals. Genauere Angaben zu diesem Test finden sich in Beispiel 1.

Mithilfe des erfindungsgemäßen Verfahrens kann erstmals die Anreicherung der Phosphopeptide mit quantitativer Ausbeute bei gleichzeitig nahezu vollständiger Reinheit erreicht werden.

Wichtig ist auch, dass das erfindungsgemäße Verfahren zusätzlich
- keine Unterscheidung macht zwischen verschiedenen Phosphoaminosäuren
- eine direkte Analyse der isolierten Phosphopeptide erlaubt
- einfach durchzuführen ist
- möglichst ohne Probenmodifikation arbeitet, also aufwändige Reaktionsfolgen zur chemischen Modifikation von Peptidgemischen und die damit verbundenen Probleme hinsichtlich der Reproduzierbarkeit und der einzusetzenden Probenmenge vermeidet, und
- auch auf nicht stoffwechselaktive Proben anwendbar ist, um klinisch relevante Proben wie etwa Gewebe von Biopsien einer Analyse zugänglich zu machen.

Die Effizienz der Anreicherung von Phosphopeptiden mittels IMAC wird in der Regel insbesondere von den folgenden Parametern beeinflußt:
- Auswahl des Trägermaterials
- Auswahl des Chelatliganden
- Auswahl der Metallionen zur Aktivierung des Chelatliganden
- Pufferbedingungen bei der Bindung der Phosphopeptide
- Pufferbedingungen bei der Elution der Phosphopeptide

Es wurde nun gefunden, dass eine spezielle Auswahl dieser Parameter eine besonders effiziente Anreicherung bezüglich Ausbeute und Reinheit des Produktes ergibt. Im folgenden werden daher zunächst die einzelnen Verfahrensschritte in allgemeiner Weise aufgeführt und anschließend die jeweiligen Parameter diskutiert.

Für die Isolierung von Phosphopeptiden mittels IMAC werden typischerweise die folgenden Verfahrensschritte durchgeführt:
a) Bereitstellen eines Trägermaterials mit Chelatligand
b) Aktivieren des Chelatliganden mithilfe von Metallionen
c) Zugabe der Phosphopeptid-haltigen Probe zum Trägermaterial in Anwesenheit eines Bindungspuffers (der Bindungspuffer kann zuvor mit der Probe und/oder dem Trägermaterial gemischt werden. Genauso kann der Bindungspuffer bereits in Schritt b) dem Trägermaterial zugesetzt werden)
d) Entfernen des Überstandes bestehend aus Bindungspuffer und nichtgebundenem Anteil der Probe
e) optional Waschen des Trägermaterials mit den gebundenen Phosphopeptiden
f) die Elution der gebundenen Phosphopeptide vom Trägermaterial

Die eluierten Phosphopeptide können dann jeder geeigneten Analyse zugeführt werden. Besonders geeignete Verfahren sind chromatographische Verfahren wie die Dünnschicht-Chromatographie, Sequenzierverfahren und/oder spektrometrische Verfahren wie insbesondere MALDI oder ESI Massenspektrometrie. Die Proben können dazu direkt oder nach vorheriger Entsalzung der Analyse zugeführt werden. Eine Entfernung der Salze des Elutionspuffers kann notwendig sein, da manche Analysenmethoden, insbesondere massenspektrometrische Methoden wie MALDI Massenspektrometrie, durch das Vorhandensein von Salzen gestört werden. Eine mögliche Methode zur Entsalzung ist z.B. eine Reversed Phase Chromatographie.

Es wurde gefunden, dass bei Verwendung des erfindungsgemäß bevorzugten Elutionspuffers auf Basis von Thiocyanat-Salzen die Probe direkt ohne vorherige Entsalzung mittels MALDI Massenspektrometrie analysiert werden kann und trotzdem hervorragende Analyseempfindlichkeiten erreicht werden. Dies war mit den bislang bekannten Elutionspuffern bei gleichzeitiger hoher Phosphopeptid-Ausbeute nicht möglich.

Diskussion der einzelnen Parameter:

### Aktivierung der Chelatliganden

Die Aktivierung der Chelatliganden des Trägermaterials erfolgt mit Übergangsmetallionen, Oxyden- und Oxydhydraten von Übergangsmetallen oder dreiwertigen Ionen von Metallen der dritten Hauptgruppe. Bevorzugt erfolgt die Aktivierung mit Eisen (III) - Ionen, besonders bevorzugt mit Zirkonium (IV)-Ionen. Dazu wird das Trägermaterial mit Chelatliganden in wässrigen Lösungen der entsprechenden Metallsalze inkubiert. Die Konzentration der Lösungen beträgt typischerweise zwischen 1 und 500 mmol/l. Die Dauer der Inkubation beträgt typischerweise zwischen 1 Minute und 12 Stunden. Beispiele für geeignete Salze sind MnCl₂, NiCl₂, CuCl₂, GaCl₃ und insbesondere FeCl₃ und ZrOCl₂.

### Bindungspuffer

Als Bindungspuffer kann jeder nach dem Stand der Technik für die IMAC von Phosphopeptiden bekannte Bindungspuffer eingesetzt werden. Beispiele hierfür sind 0,005 bis 20%ige Essigsäure oder Ameisensäure in Wasser. Bevorzugt wird erfindungsgemäß 1,5%ige Essigsäure oder Ameisensäure in Wasser als Bindungspuffer eingesetzt.

### Waschpuffer

Als Waschpuffer kann jeder nach dem Stand der Technik für die IMAC von Phosphopeptiden bekannte Waschpuffer eingesetzt werden. Beispiele hierfür sind 0,001 bis 2%ige Essigsäure oder Ameisensäure in Wasser. Bevorzugt werden erfindungsgemäß 0,1 %ige Essigsäure oder Ameisensäure in Wasser als Waschpuffer eingesetzt. Bevorzugt werden zwei oder mehrere Waschschritte durchgeführt, wobei im ersten Schritt ein rein wässriger Waschpuffer verwendet wird und im nächsten Schritt ein Waschpuffer, der einen Anteil eines organischen Lösungsmittels, wie z.B. Acetonitril oder Methanol enthält.

### Elutionspuffer

Die Elution der Phosphopeptiden von dem Träger erfolgt erfindungsgemäß mit Elutionsppuffern, die einen pH-Wert > 10, bevorzugt einen pH-Wert von ca. 10,5 aufweisen und zumindest eine oder mehrere der folgenden Komponenten enthalten:
- Alkali- Erdalkali- oder Ammoniumsalze des Thiocyanats
- Alkali- Erdalkali- oder Ammoniumsalze von Säuren der Komplexliganden Nitrito, Isocyano, Nitril, Isocyanato, Isothiocyanato, Azido, Ethylendiamin, Isonitril, Fulminato und/oder Cyano,
- Alkali- Erdalkali- oder Ammoniumsalze von Sauerstoffsäuren des Phosphors, Schwefels, Vanadiums, Rutheniums, Niobs, Tantals, Wolframs und/oder des Molybdäns
- Chelatbildner wie EDTA, EGTA oder Salicylsäure.

Überraschenderweise wurde gefunden, dass die Ausbeute der Elution der Phosphopeptide von mit Metallionen aktivierten erfindungsgemäßen Silica-Trägern mit Chelatliganden deutlich gesteigert werden kann, wenn Elutionspuffer mit stark alkalischem pH-Wert eingesetzt werden. Durch die erfindungsgemäße Elution der Phosphopeptide mit Natrium- und Ammoniumsalzen von Sauerstoffsäuren des Phosphors bei pH > 10 konnten z.B. im Falle der Verwendung von sowohl Eisen (III), Gallium (III) und Zirkonium (IV) deutlich höhere Ausbeuten erzielt werden als nach dem Stand der Technik.

Erfindungsgemäß bevorzugt ist die Elution mit Elutionspuffern, die Alkali-Erdalkali- und/oder Ammoniumsalze von Sauerstoffsäuren des Phosphors enthalten.

Weiterhin wurde gefunden, dass eine besonders effektive Elution mit Alkali-Erdalkali- und Ammoniumsalzen von Thiocyanat gelingt und diese Eluate besonders gut direkt ohne weitere Reinigung massenspektrometrisch untersucht werden können. Erfindungsgemäß besonders bevorzugte Elutionspuffer enthalten daher Alkali- Erdalkali- und/oder Ammoniumsalze von Thiocyanat.

Der Konzentrationsbereich der Salze in den Elutionspuffer liegt typischerweise zwischen 0.005 und 2 mol/l. Bevorzugt sind Konzentrationen zwischen 50 und 200 mM, besonders um ca. 100 mM.

Gegenstand der vorliegenden Erfindung ist auch ein Kit zur Anreicherung von Phosphopeptiden zumindest enthaltend Trägermaterial mit Chelatliganden auf Basis von Silica und einen Elutionspuffer, der einen pH-Wert > 10, besonders bevorzugt einen pH-Wert von ca. 10,5 aufweist und zumindest ein oder mehrere der folgenden Komponenten enthält:
- Alkali- Erdalkali- oder Ammoniumsalze des Thiocyanats
- Alkali- Erdalkali- oder Ammoniumsalze von Säuren der Komplexliganden Nitrito, Isocyano, Nitril, Isocyanato, Isothiocyanato, Azido, Ethylendiamin, Isonitril, Fulminato und/oder Cyano,
- Alkali- Erdalkali- oder Ammoniumsalze von Sauerstoffsäuren des Phosphors, Schwefels, Vanadiums, Rutheniums, Niobs, Tantals, Wolframs und/oder des Molybdäns
- Chelatbildner wie EDTA, EGTA oder Salicylsäure.

Dabei kann das Trägermaterial in aktivierter oder nicht aktivierter Form vorliegen. Bevorzugt liegt das Trägermaterial als wässrige Suspension in mit Eisen (III) oder Zirkonium (IV)-Ionen aktivierter Form vor.

In einer bevorzugten Ausführungsform enthält der Kit zusätzlich weitere Bestandteile wie bevorzugt Bindungs- und/oder Waschpuffer.

Weiterhin kann er weitere Bestandteile, wie z.B. Reagenzien zur Aktivierung des Trägermaterials enthalten.

In einer besonders bevorzugten Ausführungsform enthält der Kit ein mit Zirkonium (IV)-Ionen aktiviertes Trägermaterial in Form von Magnetit-Partikeln, die zumindest teilweise mit Silica überzogen sind und Chelatliganden der Formel Ia und/oder Ib mit R = Methyl und m = 2 tragen.

In einer weiteren bevorzugten Ausführungsform enthält der Kit als Elutionspuffer einen Puffer mit 0,005 bis 2 mol/l Ammoniumthiocyanat und einem pH-Wert > 10, besonders bevorzugt mit einem pH-Wert von ca. 10,5.

Auch ohne weitere Ausführungen wird davon ausgegangen, daß ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

Die vollständige Offenbarung aller vor- und nachstehend aufgeführten Anmeldungen, Patente und Veröffentlichungen, insbesondere der korrespondierenden Anmeldung EP 04011468.8, eingereicht am 14.05.2004, ist durch Bezugnahme in diese Anmeldung eingeführt.

### Beispiele

### 1. chromatographische Trennung dreier Peptide zur Bestimmung der Ausbeute und Reinheit der Aufreinigung

Für diesen Test wurde ein gering komplexes Modellpeptidgemisch aus drei im Retentionsverhalten hinreichend verschiedenen Peptiden benutzt. Eines dieser Peptide ist basisch, eines sauer und ein drittes ist ein Monophosphopeptid. Die Charakteristika der Peptide sind in Tabelle 1 zusammengefasst.

**Tabelle 1: Charakteristika der Modellpeptide zur quantitativen und qualitativen Analyse von Verfahren zur Phosphopeptid-Anreicherung Peptid 2 ist das einzige eingesetzte Phosphopeptid neben einem basischen (Peptid 1) und einem sauren (Peptid 3).**

| **Peptid** | **Aminosäurensequenz** | **Molekular-Gewicht** | **pl- Wert** |
|---|---|---|---|
| 1 | IFVQKCAQCHTVEK | 1633,94 g/mol | 8,06 |
| 2 | DLDVPIPGRFDRRVpSVAAE | 2192,40 g/mol | 4,93 |
| 3 | EDLIAYLK | 964,13 g/mol | 4,37 |

Der Test erlaubt somit, mit Hilfe eines direkt quantifizierbaren Signals die Abtrennung von sauren sowie basischen Peptiden von einem in der Mischung vorhandenen Phosphopeptid zu untersuchen.

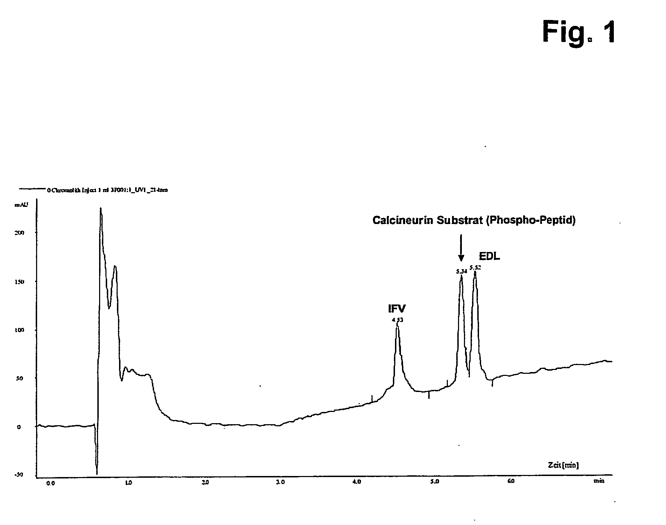

Abbildung 1 zeigt ein typisches RP-HPLC-Profil der Trennung des Modellpeptidgemisches. Alle Signale sind ausreichend getrennt, was eine eindeutige Integration der entsprechenden UV-Signale erlaubt. Unter Berücksichtigung der Standardabweichung in der Höhe von maximal 0,05 min bzw. 3 s sind die Netto- Retentionszeiten der einzelnen Peptide (siehe Tabelle 2) zudem hinreichend unterschiedlich und reproduzierbar, so dass eine eindeutige Zuordnung der Peptide in Chromatogrammen umfangreicher Versuchsreihen möglich ist. Werden die Modell- Peptide als Kalibrationsstandards vermessen, entsprechen die Flächen dieser Peaks zur Berechnung von Ausbeute und Reinheit des Phosphopeptides 100 %.

**Tabelle 2: Die Retentionszeiten der Modellpeptide sind hinreichend verschieden und reproduzierbar, um eine eindeutige Zuordnung und Integration der Signale zu erlauben. Die Tabelle zeigt die Mittelwerte der Retentionszeiten der Modell- Peptide aus drei voneinander unabhängig durchgeführten Messungen.**

| **Peptid** | **Netto-Retentionszeit [min]** | | | **Mittelwert [min]** | **Standardabweichung [min]** |
|---|---|---|---|---|---|
| 1 | 4,53 | 4,55 | 4,47 | **4,52** | 0,04 |
| 2 | 5,34 | 5,25 | 5,25 | **5,28** | 0,05 |
| 3 | 5,42 | 5,51 | 5,43 | **5,45** | 0,05 |

Die Trennungen erfolgen auf einer Chromolith^{®} Performance RP 18e Säule, welche an eine ÄKTA Explorer Chromatographie-Einheit angeschlossen ist. Es wird ein linearer Gradient von Acetonitril in Wasser vor einem Hintergrund von 0,1 % TFA bei einer Flussrate von 3 ml/min eingesetzt. Die Eluenten sind A: Wasser mit 0,1 % TFA und B: 80% Acetonitril und 0,1% TFA in Wasser.

| | |
|---|---|
| Äquilibrierung: | 6 ml bei 5% B |
| Gradient: | 5-100% B bei Erhöhung um 9,5% B/min anschließend Reduktion auf 5% B in 1 min |
| Reäquilibrierung: | 6 ml bei 5% B |

### 2. Synthese der erfindungsgemäßen Trägermaterialien mit Chelatligand

### 1. Herstellung von epoxymodifizierten magnetischen Silica-Partikeln:

5 g MagPrep^{®} Silica HS Partikel (Merck KGaA, Deutschland) werden mehrfach mit VE-Wasser salzfrei gewaschen und anschließend in 100 ml VE-Wasser suspendiert. Die Suspension wird in einem mit KPG-Rührer, Rückflusskühler und Tropftrichter ausgestatteten Dreihalskolben gerührt. In der Magnetpartikelsuspension werden 0,01 mol Natriumacetat aufgelöst.

Anschließend werden innerhalb 15 min 1,25 g Glycidyloxypropyltrimethoxysilan, gelöst in 8,3 ml Isopropanol, zugetropft. Das Gemisch wird auf 80°C erhitzt und 3 h bei dieser Temperatur gerührt. Nach dem Abkühlen werden die Magnetpartikel 5 mal mit je 100 ml VE-Wasser gewaschen. Sollen die Partikel gleich weiter umgesetzt werden, können sie in der wässrigen Suspension verbleiben. Ansonsten werden sie mehrfach mit Aceton wasserfrei gewaschen und dann 1,5 h lang im Vakuum bei 50°C getrocknet.

### 2. Herstellung von IDA-modifizierten magnetischen Silica-Partikel:

In 100 ml einer 5%igen Suspension der nach Schritt 1 hergestellten Epoxy-aktivierten Partikel werden 1,5 g Iminodiessigsäure und 0,8 g Natriumacetat gelöst. Mit ein paar Tropfen verdünnter Natronlauge wird der pH auf 9 eingestellt und das Gemisch 1 h lang bei 70°C gerührt. Nach dem Abkühlen werden die Partikel 5 mal mit je 100 ml VE-Wasser gewaschen. Sie werden als 5%ige Suspension in VE-Wasser gelagert.

### 3. Herstellung von Methyl-EDDA-modifizierten magnetischen Silica-Partikeln:

100 ml einer 5%igen wässrigen Suspension der nach Schritt 1 hergestellten Epoxy-aktivierten Partikel werden in der oben beschriebenen Apparatur 1 h lang mit 2,65 g N-Methyl-ethylendiamin bei 65°C gerührt. Nach dem Abkühlen werden die Partikel 5 mal mit je 100 ml VE-Wasser gewaschen. In der resultierenden Suspension (100 ml) werden 5,5 g Bromessigsäure gelöst. Nach Zugabe von ca. 0,8 g Natriumacetat wird der pH-Wert mit verdünnter NaOH auf 8 eingestellt und die Suspension über Nacht bei Raumtemperatur gerührt. Anschließend werden die Partikel mehrmals mit VE-Wasser gewaschen.

### 4. Herstellung von Ethyl-EDDA-modifizierten magnetischen Silica-Partikeln:

Die Herstellung erfolgt analog Schritt 3, jedoch mit 3,15 g N-Ethylethylendiamin.

### 5. Herstellung von Hydroxyethyl-EDDA-modifizierten magnetischen Silica-Partikeln:

3,12 g 2-(2-Aminoethylamino)-ethanol werden mit VE-Wasser auf 25 ml aufgefüllt (ca. 1 M). Die Lösung wird mit HCI auf pH 10 eingestellt. In dieser Lösung werden 1 g Aceton-getrocknete Epoxy-aktivierte Partikel suspendiert. Man lässt 7 h lang bei Raumtemperatur schütteln. Anschließend wird fünfmal mit VE-Wasser gewaschen und bei 50 °C im Vakuum für 1,5 h getrocknet.

### 6. Herstellung von epoxymodifizierten Silicapartikeln:

50 g LiChrospher^{®} Si 300, 15 - 40 µm Partikel (Merck KGaA, Deutschland) werden in 600 ml 0,1 molarer Natriumacetatlösung suspendiert. Die Suspension wird in einem mit KPG-Rührer, Rückflusskühler und Tropftrichter ausgestatteten Dreihalskolben gerührt. Anschließend werden innerhalb 25 min 80 g Glycidyloxypropyltrimethoxysilan, gelöst in 400 ml Isopropanol, zugetropft. Das Gemisch wird auf 80°C erhitzt und 2 h bei dieser Temperatur gerührt. Nach dem Abkühlen wird das Kieselgel über eine Glasfilterfritte abgesaugt und zunächst 5 mal mit je 200 ml VE-Wasser, dann 2 mal mit je 200 ml Isopropanol gewaschen. Das Material wird 24 h lang bei 50°C im Vakuum getrocknet.

### 7. Herstellung von Methyl-EDDA-modifizierten Silicapartikeln:

25 g der nach Beispiel 6 hergestellten Epoxy-aktivierten Kieselgelpartikel werden in 300 ml VE-Wasser suspendiert und in der oben beschriebenen Apparatur 1,5 h lang mit 75 ml N-Methyl-ethylendiamin bei 65°C gerührt. Nach dem Abkühlen wird das Kieselgel über eine Glasfilterfritte abgesaugt und 5 mal mit je 200 ml VE-Wasser gewaschen. Anschließend wird das feuchte Kieselgel in 600 ml 0,1 molarer Natriumacetatlösung resuspendiert. Es werden 17,6 g Bromessigsäure zugegeben und der pH-Wert der Suspension mit 10%iger Natronlauge auf 8,7 eingestellt. Das Gemisch wird 20 h lang bei Raumtemperatur gerührt. Anschließend wird das Kieselgel über eine Glasfilterfritte abgesaugt und mehrmals mit VE-Wasser nachgewaschen.

### 8. Aktivierung der Chelat-modifizierten Partikel mit Metallionen:

5 mmol Metallsalz (z.B. MnCl₂, NiCl₂, FeCl₃, CuCl₂, GaCl₃, ZrOCl₂) werden in 20 ml VE-Wasser gelöst. Gegebenenfalls wird der pH-Wert durch Zugabe einiger Tropfen verdünnter Natronlauge, bzw. Salzsäure auf 7 eingestellt. Anschließend wird die Lösung mit einer Suspension aus 1 g chelat-modifizierten Partikeln (Herstellung entsprechend Schritt 2-5 und 7) in 20 ml VE-Wasser vermischt und 1 h lang bei Raumtemperatur geschüttelt. Die Partikel werden zehnmal mit VE-Wasser gewaschen und als 5%ige wässrige Suspension gelagert.

### 3. Protokoll zur Durchführung des erfindungsgemäßen Anreicherungsverfahrens

In seiner bevorzugten Ausführungsform wird das erfindungsgemäße Verfahren zur Isolierung von Phosphopeptiden nach dem folgenden Protokoll durchgeführt. Die generellen Verfahrensparameter, d.h. die Abfolge der Verfahrensschritte, ihre Dauer etc. ist auch auf die Durchführung des erfindungsgemäßen Verfahrens mit anderen Reagenzien (z.B. Aktivierung mit anderen Metallionen, Einsatz anderer Puffer oder Trägermaterialien) übertragbar.

Als Trägermaterial'werden magnetische Silicapartikel, modifiziert mit EDDA-methyl und aktiviert mit Zirkonium (IV) verwendet. Eine 5 %-ige (w/v) Suspension der Partikel in Wasser findet Verwendung gemäß dem unten dargestellten Protokoll.

Als Elutionspuffer wird 100 mM Ammoniumthiocyanat, pH 10.5 in Wasser, eingesetzt.

Bindungs- und Waschpuffer sind wie folgt zusammengesetzt:
○ Bindungspuffer :1,5 % (v/v) Essigsäure in Wasser
○ Waschpuffer 1: 0,1 % Essigsäure in Wasser
○ Waschpuffer 2: 0,1 % Essigsäure/30 % Acetonitril in Wasser

### Äquilibrierung des Trägermaterials

1. Mische den Inhalt des Behälters mit den Partikeln gründlich um eine homogene Suspension der Partikel zu erhalten
2. In einem Mikrozentrifugenröhrchen werden 50 µl Partikel-Suspension vorgelegt und zur Abscheidung der Partikel für 1 min bei RT in einem Magnetseparator (z.B. Dynal MPC®-S) inkubiert. Der Überstand wird verworfen.
3. Das Mikrozentrifugenröhrchen wird aus dem Magnetseparator entfernt und die Partikel in 200 µl Wasser resuspendiert.
4. Zur Abscheidung der Partikel wird erneut für 1 min bei RT in einem Magnetseparator (z.B. Dynal MPC®-S) inkubiert. Der Überstand wird verworfen.
5. Schritt drei und vier werden einmal wiederholt.
6. Das Mikrozentrifugenröhrchen wird aus dem Magnetseparator entfernt und die Partikel in 100 µl Bindungspuffer resuspendiert.
7. Zur Abscheidung der Partikel wird erneut für 1 min bei RT in einem Magnetseparator (z.B. Dynal MPC®-S) inkubiert. Der Überstand wird verworfen.

### Vorbereitung der Phosphopeptid-Probe

1. In einem Mikrozentrifugenröhrchen werden 10-20 µl Probelösung vorgelegt und mit 90 µl Bindungspuffer verdünnt.

### Anreicherung von Phosphopeptiden

1. Die Partikel werden in der verdünnten Probe resuspendiert und die Suspension für 10 min bei RT unter Schütteln inkubiert.
2. Zur Abscheidung der Partikel wird für 1 min bei RT in einem Magnetseparator (z.B. Dynal MPC®-S) inkubiert. Der Überstand wird verworfen.
3. Die Partikel werden in 100 µl Waschpuffer 1 resuspendiert und anschließend zur Abscheidung der Partikel wird für 1 min bei RT in einem Magnetseparator (z.B. Dynal MPC®-S) inkubiert. Der Überstand wird verworfen.
4. Schritt 3 wird wiederholt
5. Die Partikel werden in 100 µl Waschpuffer 2 resuspendiert und anschließend zur Abscheidung der Partikel wird für 1 min bei RT in einem Magnetseparator (z.B. Dynal MPC®-S) inkubiert. Der Überstand wird verworfen.
6. Schritt 5 wird wiederholt
7. Die Partikel werden durch Zentrifugation für 1 min bei RT und 1000 - 2000 x g isoliert. Der Überstand wird verworfen.
8. Die Partikel werden in 25 µl Elutionspuffer resuspendiert und die Suspension für 10 min bei RT unter Schütteln inkubiert.
9. Die Partikel werden durch Zentrifugation für 2 min bei RT und 10.000 x g isoliert.
10. Der Überstand enthält die angereicherten Phosphopeptide.

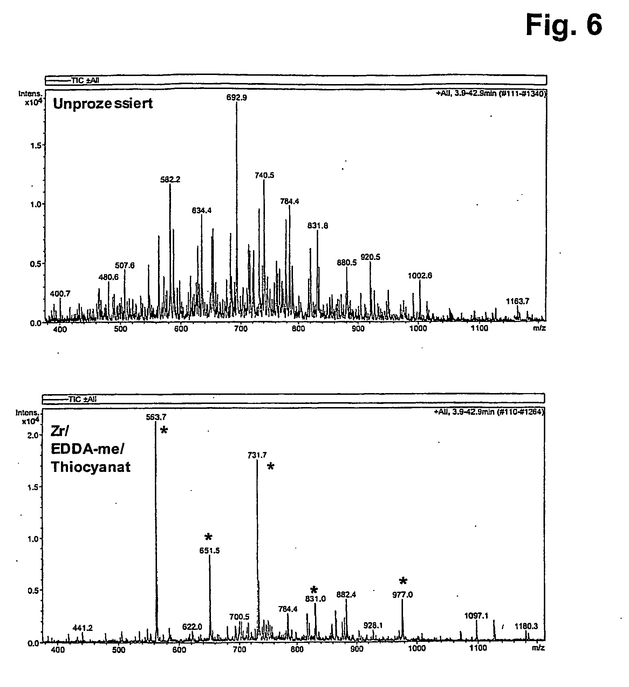

Abbildung 6 zeigt das Resultat der Anreicherung von Phosphopeptiden aus einer komplexen Mischung mit der oben dargestellten bevorzugten Ausführungsform des erfindungsgemäßen Verfahrens. Ein äquimolares Gemisch aus tryptischen Peptiden von Rinderserumalbumin, Histon TypIIB1 und alpha-Casein sowie einem synthetischen serinphosphorylierten Monophosphopeptid (2 x 10⁻¹⁰ mol je Peptid) wurde zusätzlich mit 2 x 10⁻¹¹ mol eines synthetischen tyrosinphosphorylierten Peptides versetzt. Die Probe enthielt demnach etwa 125 erwartete Peptide, davon zwei erwartete serinphosphorylierte Monophosphopeptide sowie zusätzlich ein tyrosinphosphoryliertes Monophosphopeptid, welches mit 10 % der molaren Menge aller anderen Peptide vorlag. Das mehrfach phosphorylierte Peptid des alpha-Caseins konnte unter den angewendeten Einstellungen des Massenspektrometers nicht detektiert werden. Dargestellt sind repräsentative Spektren der unprozessierten Probe sowie des Eluates nach der Affinitätsanreicherung von Phosphopeptiden mit dem erfindungsgemässen Verfahren, die Phosphopeptidsignale sind in Tabelle 8 erläutert.

**Tabelle 8: Zuordnung der Phosphopeptidsignale nach der Anreicherung von synthetischen und natürlichen phosphorylierten Peptiden aus einer komplexen Mischung.**

| **Nr.** | **m/z** | **Aminosäuren-Sequenz** | **Ion** | **Protein / Peptid** |
|---|---|---|---|---|
| **1** | 563,7 | DRVpYIHPF | [M+2H]⁺² | p-Angiotensin |
| **2** | 651,5 | YKVPQLEIVPNpSAEER | [M+3H]⁺³ | α- S1- Casein |
| **3** | 731,7 | DLDVPIPGRFDRRVpSVAAE | [M+3H]⁺³ | Calcineurin-Substratpeptid |
| **4** | 831 | VPQLEIVPNpSAEER | [M+2H]⁺² | α- S1- Casein |
| **5** | 977 | YKVPQLEIVPNpSAEER | [M+2H]⁺² | α- S1- Casein |

Alle erwarteten Phosphopeptide werden deutlich angereichert und sind die Hauptsignale des Spektrums. Die gleichzeitige Anreicherung von serin- und tyrosinphosphorylierten Peptiden zeigt zudem an, daß das Verfahren nicht zwischen den verschiedenen Phosphoaminosäuren unterscheidet. Das tyrosinphosphorylierte Peptid wird mit einer m/z = 563.7 für das Ion [M+2H]⁺² detektiert und ist deutlich angereichert gegenüber den nicht phosphorylierten Peptiden in der unprozessierten Probe. Desweiteren repräsentieren die Signale der Phosphopeptide die Hauptsignale des Spektrums, während verbliebene Verunreinigungen nahezu nicht mehr detektiert werden können.

Dieses Ergebnis zeigt, daß mit Hilfe des erfindungsgemäßen Verfahrens zur Anreicherung gleichzeitig Peptide mit verschiedenen Phosphoaminosäuren angereichert werden können. Desweiteren wurde gezeigt, daß auch Phosphopeptide, die in substöchiometrischer Menge in einem Gemisch nicht phosphorylierter Peptide vorliegen, für die Analyse angereichert und nahezu quantitativ von den Verunreinigungen abgetrennt werden können.

### 4. Anreicherung von Phosphopeptiden durch IMAC mit IDA-modifizierten Chromatographiematerialien

Die Anreicherung von Phosphopeptiden durch IMAC mit polymeren IDA-modifizierten Trägermaterialien wurde mit Hilfe des in Beispiel 1 beschriebenen Tests analysiert. Zusätzlich wurden IDA-modifizierte magnetische Silicapartikel hergestellt (entsprechend Beispiel 2) und unter gleichen Bedingungen eingesetzt (Tabelle 3). Als Metallionen wurden exemplarisch Ga(III), Fe(III) und Zr(IV) eingesetzt.

Die Auswertung bestätigt, daß gemäß dem dokumentierten Stand der Technik keine gleichzeitige quantitative Ausbeute und Reinheit im Sinne der vorliegenden Erfindungsmeldung erzielt werden kann. Die beste Reinheit (85 % bei gleichzeitig nur 30 % Ausbeute) wurde unter Verwendung des dreiwertigen Gallium-Ions in Verbindung mit einem polymeren Trägermaterial erzielt, die höchste Ausbeute (64 % bei gleichzeitig nur 44 % Reinheit) unter Verwendung des vierwertigen Zirkonium-Ions in Verbindung mit einem polymeren Trägermaterial.

**Tabelle 3: Ausbeute und Reinheit bei der Anreicherung von Phosphopeptiden durch IMAC nach dem Stand der Technik mit IDA-modifizierten Chromatographiematerialien.**

| **Material** | **Modifikation** | **Metallion** | **Elution** | **Ausbeuten [%]** | **Reinheit [%]** |
|---|---|---|---|---|---|
| Polymer | IDA | Gallium (III) | 0.1 N NH₄OH, pH 11.2 | 30 | 85 |
| Polymer | IDA | Gallium (III) | 0.2 M Na₃PO₄, pH 8.4 | n.d. | n.d. |
| | | | | | |
| Polymer | IDA | Eisen (III) | 0.1 N NH₄OH, pH 11.2 | 2 | 37 |
| Polymer | IDA | Eisen (III) | 0.2 M Na₃PO₄, pH 8.4 | 44 | 59 |
| | | | | | |
| Polymer | IDA | Zirkonium (IV) | 0.1 N NH₄OH, pH 11.2 | 64 | 44 |
| Polymer | IDA | Zirkonium (IV) | 0.2 M Na₃PO₄, pH 8.4 | 34 | 40 |
| | | | | | |
| Silica | IDA | Gallium (III) | 0.1 N NH₄OH, pH 11.2 | 21 | 82 |
| | | | | | |
| Silica | IDA | Eisen (III) | 0.1 N NH₄OH, pH 11.2 | 34 | 86 |
| | | | | | |
| Silica | IDA | Zirkonium (IV) | 0.1 N NH₄OH, pH 11.2 | 51 | 79 |

Auch die Verwendung der IDA-modifizierten magnetischen Silicapartikel in Kombination mit nach dem Stand der Technik durchgeführten Methoden zur Bindung und Elution der Phosphopeptide bringt nur wenig Verbesserung. Insbesondere die Ausbeute liegt bei ca. 50% und weniger. Die Ergebnisse dieser Versuche zeigen, dass bislang kein Verfahren bekannt ist, dass quantitative Reinheit und Ausbeute erreicht. Mit Silica-Trägern konnte im Vergleich zu polymeren Trägern eine relative Verbesserung der erzielten Reinheit beobachtet werden, die erreichbare Ausbeute war jedoch unverändert und betrug maximal etwa 50%.

### 5. Analyse der Anreicherung von Phosphopeptiden nach dem erfindungsgemäßen Verfahren

Tabelle 4 zeigt zunächst die Anreicherung von Phosphopeptiden mit dem erfindungsgemäßen Verfahren (analog Beispiel 3) unter Verwendung IDAmodifizierter magnetischer Silica-Partikel. Zum Vergleich sind nochmals Daten zur Elution mit Hydroxyd-Puffer nach dem Stand der Technik angegeben.

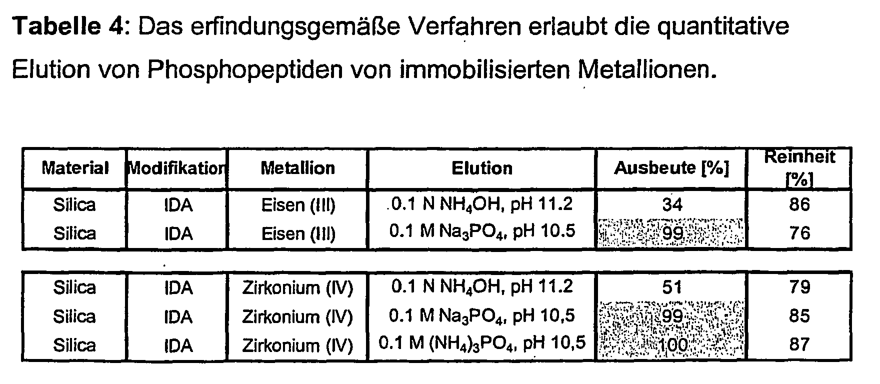

Die Tabelle zeigt deutlich, dass das erfindungsgemäße Verfahren hervorragende Ausbeuten bei sehr guter Reinheit ermöglicht.

Zur Evaluierung der erfindungsgemäßen Oberflächenmodifikation EDDA-Methyl wurde eine neuartige Silica-Oberfläche gemäß Beispiel 2 hergestellt und durch den in Beispiel 1 beschriebenen Test hinsichtlich der Ausbeute und Reinheit des isolierten Phosphopeptides evaluiert.

Die Anreicherung erfolgte nach dem Verfahren gemäß Beispiel 3.

Tabelle 5 enthält exemplarisch Daten für die Oberflächenmodifikation EDDA-Me in Kombination mit den Metallen Eisen (III) und Zirkonium (IV).

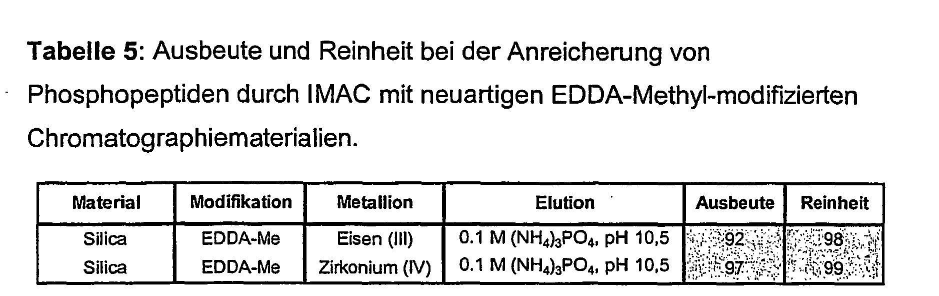

Die Ergebnisse zeigen, dass mit der erfindungsgemäßen Oberflächenmodifikation EDDA-Me mit R = Methyl besonders effiziente Anreicherungen gelingen. Mit EDDA-Me-modifizierten magnetischen Silicapartikeln in Kombination mit der erfindungsgemäß bevorzugten Elutionsmethode wurde sowohl bei der Verwendung von Eisen (III) als auch von Zirkonium (IV) gleichzeitig quantitative Ausbeute und Reinheit erreicht. Damit konnte im Vergleich zu IDA-modifizierten magnetischen Silicapartikeln (Tabelle 4) nochmals eine Erhöhung der Selektivität der Isolierung von Phosphopeptiden durch die Verwendung der neuartigen EDDA-Me-modifizierten magnetischen Silicapartikel erzielt werden. Im Falle des Eisen (III) konnte z.B. die Reinheit des isolierten Phosphopeptides von 76 % auf 98 % gesteigert werden und im Falle von Zirkonium (IV) von 87 % auf 99 %.

### 6. ESI-LC-Massenspektrometrie-Analyse der IMAC-Anreicherung von Phosphopeptiden mithilfe des erfindungsgemäßen Trägetmaterials mit dem vierzähnigen Chelatbildner EDDA-Me

Alle bisherigen Analysen wurden mit einem gering komplexen Peptidgemisch durchgeführt, um das erfindungsgemäße Verfahren durch gleichzeitige quantitative und qualitative Analyse evaluieren zu können. In der Regel sind die Proben jedoch, wie einleitend dargestellt, komplexer und sie enthalten Phosphopeptide in Gegenwart einer Vielzahl nichtphosphorylierter Peptide.

Zur Evaluierung der Selektivität des erfindungsgemäßen Verfahrens zur Phosphopeptid-Anreicherung mittels des neuartigen Chelatbildners EDDA-Methyl im Vergleich zu IDA wurde ein komplexes Testgemisch durch den tryptischen Verdau von äquimolaren Mengen der drei Proteine alpha-S1-Casein, Rinderserumalbumin und Histon TypIIB1 hergestellt. Dieses Peptidgemisch enthält bei angenommener vollständiger tryptischer Spaltung ein Monophosphopeptid des alpha-Caseins in einem Hintergrund aus 125 nicht phosphorylierten Peptiden. Die Analyse erfolgt exemplarisch durch LC/ESI-Massenspektrometrie. Das mehrfach phosphorylierte Peptid des alpha-S1-Caseins ist durch die verwendete Einstellung des Mssenspektrometers der Analyse nicht zugänglich. Im Gegensatz zum in Beispiel 1 beschriebenen Testverfahren erlaubt die Massenspektrometrie allerdings keine quantitative Interpretation des Ergebnisses. Die relative Intensität des Ionensignals eines bestimmten Peptides hängt ausser von seiner relativen Abundanz von einer Reihe weiterer Parameter wie der lonisierbarkeit des Peptides ab.

Die hohe Empfindlichkeit der Massenspektrometrie macht diese dennoch zum Detektionssystem der Wahl bei der Analyse von Phosphopeptiden. Desweiteren erlaubt die hohe Sensitivität der Massenspektrometrie sehr gute Aussagen über die Selektivität eines bestimmten Verfahrens zur Phosphopeptidanreicherung, wobei die relative Intensität des Phosphopeptid-Signals aus oben genannten Gründen jedoch kein quantifizierbares Kriterium für die Reinheit der Probe darstellt.

Eine Menge des Testgemisches, die 2 x 10⁻¹⁰ mol jeden Peptides entspricht, wurde mit dem erfindungsgemäßen Verfahren zur Phosphopeptid-Anreicherung prozessiert, wobei entweder ein IDAmodifizierter Silicaträger oder ein EDDA-Me-modifizierter Silicaträger gemäß Beispiel 2 eingesetzt wurde. Das Anreicherungsverfahren wurde gemäß Beispiel 3 durchgeführt, wobei der Elutionspuffer statt Ammoniumthiocyanat Ammoniumphosphat enthielt.

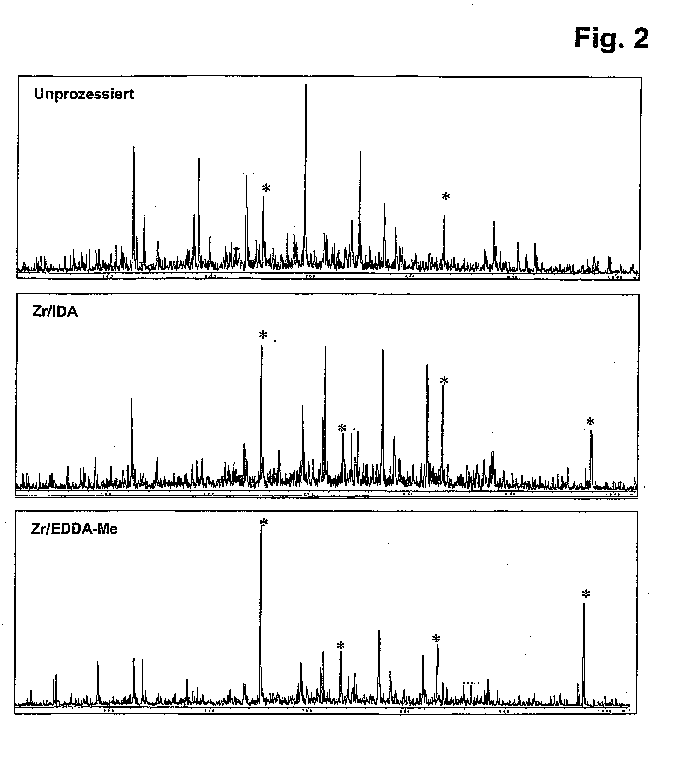

Wie Abbildung 2 zeigt, kann eine höhere Selektivität des neuartigen Chelatbildners EDDA-Me im Vergleich zu IDA in Übereinstimmung zu den oben beschriebenen Resultaten auch mit einer komplexen Probe dokumentiert werden. Die detektierten Signale von Phosphopeptiden sind in Tabelle 6 zusammengefasst.

**Tabelle 6: Zuordnung der Phosphopeptidsignale nach der Anreicherung von phosphorylierten Peptiden aus einer komplexen Mischung.**

| **Nr.** | **m/z** | **Aminosäuren-Sequenz** | **Ion** | **Protein** |
|---|---|---|---|---|
| **1** | 651,4 | YKVPQLEIVPNpSAEER | [M+3H]⁺³ | α- S1- Casein |
| **2** | 734,3 | TVDMEpSTEVFTV | [M+2H]⁺² | α- S2 Casein |
| **3** | 831,4 | VPQLEIVPNpSAEER | [M+2H]⁺² | α- S1- Casein |
| **4** | 977 | YKVPQLEIVPNpSAEER | [M+2H]⁺² | α- S1- Casein |

Überraschenderweise werden drei verschiedene Phosphopeptide detektiert, wobei zwei davon (entsprechend den Signalen 1/4 und 3) durch unvollständige tryptische Spaltung des alpha-Caseins erklärt werden und dieselbe Phosphorylierungsstelle des Proteins enthalten. Das dritte Phosphopeptid (entsprechend dem Signal 2) entspricht einem hierzu sequenzhomologen Peptid aus dem Protein alpha-S2-Casein, welches möglicherweise als Verunreinigung in der verwendeten Charge des alpha-S1-Caseins vorlag.

Sowohl unter Verwendung der neuartigen Oberflächenmodifikation EDDA-Methyl wie auch unter Verwendung des dreizähnigen Chelatbildners IDA können gute Ergebnisse erzielt werden. Das erfindungsgemäße Verfahren zur Phosphopeptid-Anreicherung unter Verwendung der neuartigen Oberflächenmodifikation EDDA-Methyl erlaubt jedoch eine noch höhere Selektivität bei der Anreicherung von Phosphopeptiden aus komplexen Proben als mit dem dreizähnigen Chelatbildner IDA.

### 7. direkte Probenanalyse mittels MALDI-TOF-Massenspektrometrie

Eine erfolgversprechende Methode zur Phosphopeptid-Anreicherung aus komplexen Proben sollte eine direkte Analyse der isolierten Phosphopeptide insbesondere durch MALDI-Massenspektrometrie erlauben. MALDI-Massenspektrometrie ist eine sehr sensitive Analysetechnik, die in der Regel empfindlicher ist als die ESI-LC/MS, jedoch stärker durch ionische Komponenten in der Probe, wie etwa durch Salze, beeinflußt wird.

Wie oben beschrieben, wird zum Erreichen einer guten Ausbeute bei der Elution der Phosphopeptide von immobilisierten Metallionen gemäß dem erfindungsgemäßen Verfahren bevorzugt die kompetitive Elution durch Erdalkali- und Ammoniumsalze der ortho-Phosphorsäure, des Hydrogen- oder des Dihydrogenphosphates bei pH > 10 eingesetzt: Nach dem Stand der Technik können ionisch verunreinigte Proben durch zusätzliche, schlecht reproduzierbare, Verfahrensschritte wie eine Probenentsalzung, in der Regel durch *reversed phase*-Reinigung der Peptide, vor der Analyse aufgearbeitet werden. Besonders vorteilhaft wäre jedoch ein Verfahren, bei dem auf diese zusätzlichen Aufarbeitungsschritte verzichtet werden kann. Zur Evaluierung der Kompatibilität des erfindungsgemäßen Verfahrens zur Anreicherung von Phosphoproteinen mit der direkten Analyse durch MALDI-Massenspektrometrie wurde ein Peptidgemisch eingesetzt, welches ein Monophosphopeptid mit einer erwarteten Masse [M+H]⁺ von 2193,4 Da aufweist. Das Peptid gemisch wurde mit dem erfindungsgemäßen Verfahren gemäß Beispiel 3 unter Verwendung verschiedener Elutionspuffer und der neuartigen EDDA-Me-modifizierten magnetischen Silicapartikel angereichert. Alle Proben wurden direkt ohne *reversed phase*-Reinigung der Peptide durch MALDI-Massenspektrometrie analysiert.

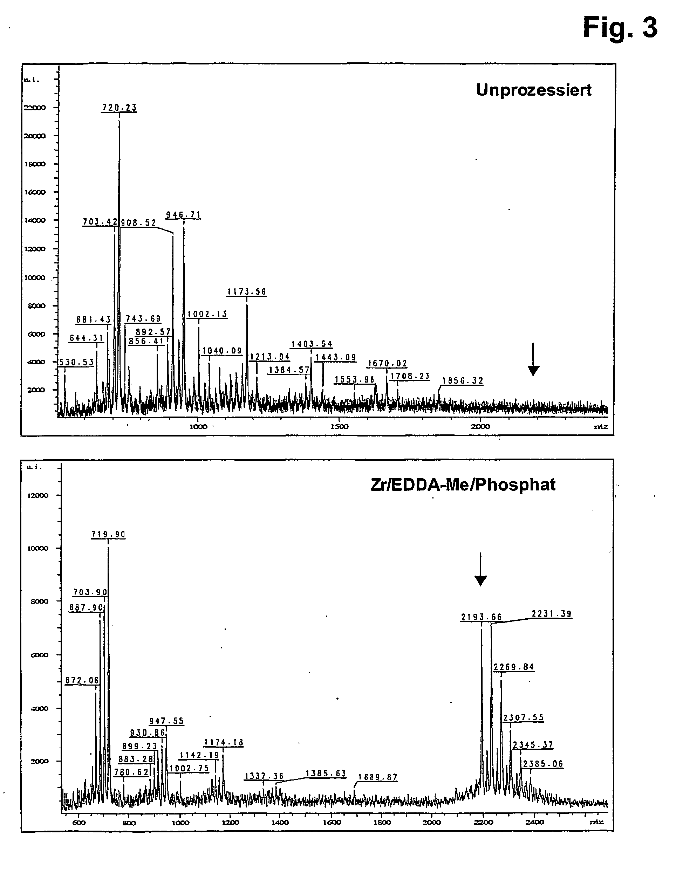

Abbildung 3 A zeigt das MALDI-MS-Spektrum der unprozessierten Probe vor der Anreicherung. Das Phosphopeptid konnte nicht detektiert werden. Nach der erfindungsgemäßen Anreicherung unter Verwendung eines Ammoniumphosphat-haltigen Elutionspuffers hingegen konnte das Phosphopeptid klar detektiert werden mit m/z = 2193.66, jedoch wurden zusätzliche Massen detektiert, die nicht durch Massen von in der Probe vorhandenen Peptide erklärt werden können (Abbildung 3B). Möglicherweise sind diese Signale Artefakte, welche durch die in der Probe vorhandenen Phosphationen verursacht wurden.

Eine wesentliche Verbesserung stellt daher die Verwendung des erfindungsgemäß bevorzugten Elutionspuffers mit Salzen der Thiocyanosäure dar.

Tabelle 7 zeigt, dass sowohl bei Verwendung von Elutionspuffern mit dem Ammoniumsalz der ortho-Phosphorsäure wie auch bei Einsatz eines Elutionspuffers mit dem Ammoniumsalz der Thiocyanosäure hervorragende Ergebnisse bei der Anreicherung erhalten werden. Allerdings ist dies überraschenderweise nur bei pH-Werten von größer 10 bis 10,5 möglich, in schwach saurem pH-Wert wurden z.B. nur 3 % Ausbeute erreicht.

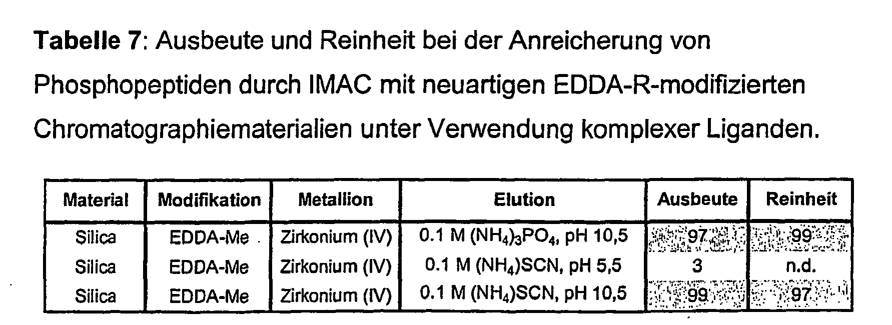

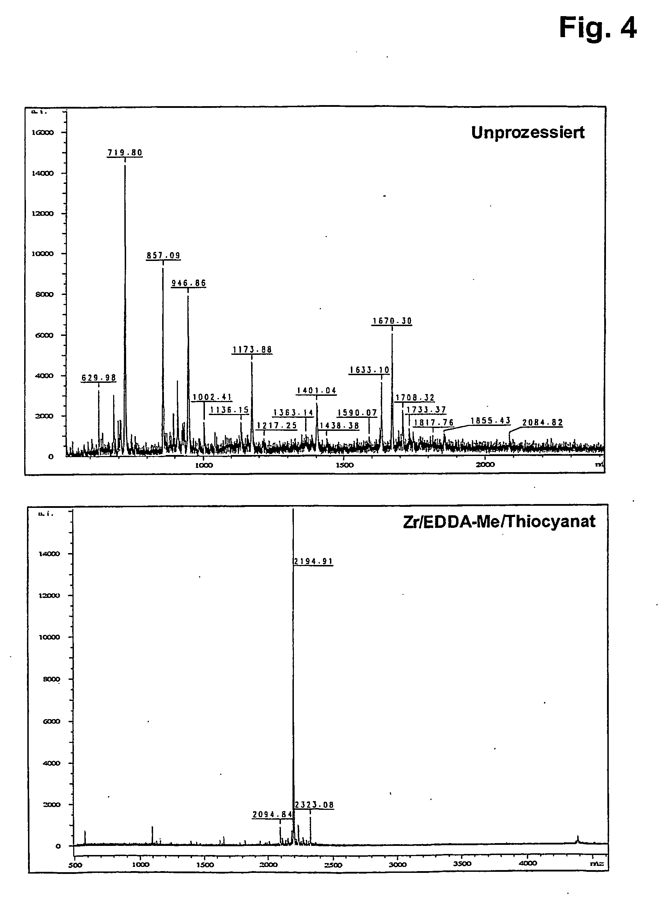

Daraufhin wurde die Kompatibilität der erfindungsgemäßen Elution von Phosphopeptiden mit alkalischen Lösungen von Komplexliganden mit der MALDI-Massenspektrometrieanalyse exemplarisch im Falle von Ammonium-thiocyanat, pH 10.5 wie oben beschrieben untersucht. Alle Proben wurden wiederum direkt ohne *reversed phase*-Reinigung der Peptide untersucht. Wie Abbildung 4 zeigt, konnte das Phosphopeptid ohne vorherige Anreicherung nicht detektiert werden. Nach der erfindungsgemäßen Anreicherung hingegen konnte das Phosphopeptid klar als einzig vorhandenes Signal detektiert werden mit m/z = 2194.91. Im Gegensatz zur Verwendung von Phosphatsalzen zur Elution (vergleiche Abb. 3) wurden nach der Elution des Phosphopeptides dem Thiocyanatsalz keine unerklärbaren Störsignale und Artefakt-Peaks detektiert.

Das erfindungsgemäße Verfahren zur Anreicherung von Phosphopeptiden unter Verwendung von neuartigen EDDA-Me-modifizierten magnetischen Silicapartikeln und von komplexen Liganden wie dem Thiocyanat-Anion zur Elution erlaubt demnach erstmals gleichzeitige quantitative Reinheit und Ausbeute bei direkter Kompatibilität mit der MALDI-Massenspektrometrie.

### 8. Vergleich des erfindungsgemäßen Verfahrens mit dem Stand der Technik

Zum Vergleich des erfindungsgemäßen Verfahrens zur Anreicherung von Phosphoproteinen mit zwei kommerziell erhältlichen, gemäß dem Stand der Technik optimierten Verfahren wurde ein Gemisch aus drei Peptiden (beschrieben in Beispiel 1) eingesetzt. Die Probe wurde nach dem erfindungsgemäßen Verfahren (entsprechend Beispiel 3) oder nach den Vorschriften gemäß dem Stand der Technik prozessiert und die entsprechenden Eluate durch LC-ESI-Massenspektrometrie analysiert.

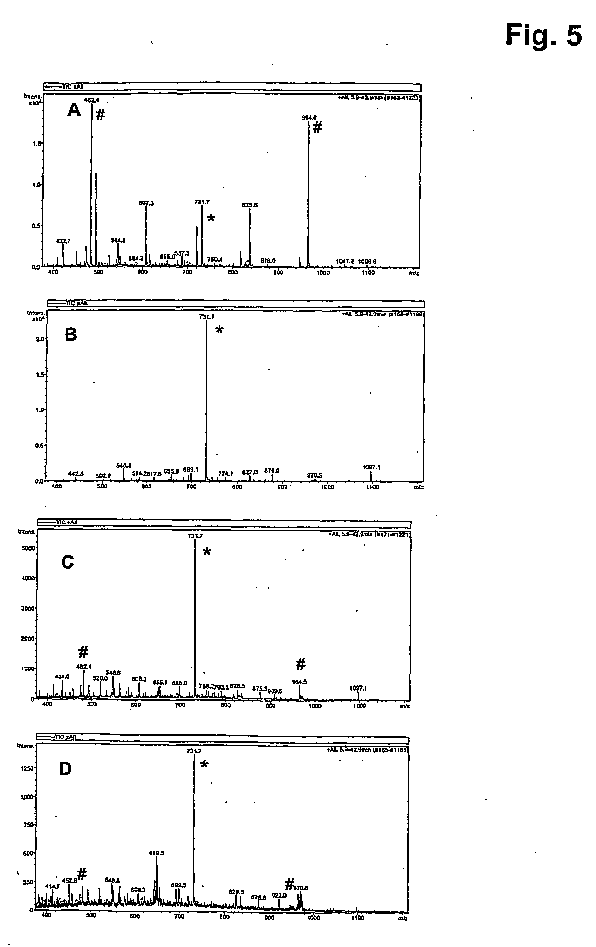

In Abbildung 5 sind repräsentative Spektren der unprozessierten Probe (A) sowie der Eluatfraktionen des erfindungsgemäßen Verfahrens (B) sowie der Verfahren nach dem Stand der Technik dargestellt (C, D). Das Abbildung 5 C entsprechende Verfahren verwendet einen vierzähnigen, NTA-analogen Chelatbildner und Eisen (III) (PHOS-Select Iron affinity gel der Firma Sigma), das Abbildung 5 D entsprechende Verfahren Gallium (III) in Kombination mit dem dreizähnigen Chelatbildner IDA (Phosphopeptide Isolation Kit der Firma Pierce). Beide Verfahren benutzen polymere Träger und schlagen die Verwendung von Ammoniumhydroxyd zur Elution gebundener Peptide vor.

Die Position des Signals des Phosphopeptides (DLDVPIPGRFDRRVpSVAAE, m/z = 731,3 [M+3H]⁺³) in den Spektren in Abbildung 5 ist durch ein Sternchen markiert, die Position der Signale zweier unterschiedlich geladener Ionen des sauren Peptides durch eine Raute.

Nur mit dem erfindungsgemäßen Verfahren wird das Phosphopeptid mit quantitativer Reinheit isoliert und kann mit einer Signalintensität von 2.3 x 10⁴ AU nachgewiesen werden. Das saure Peptid ist im Eluat des erfindungsgemäßen Verfahrens nicht nachweisbar. Beide kommerziell erhältlichen Verfahren nach dem Stand der Technik hingegen zeigen eine deutlich geringere relative Intensität des Phosphopeptid-Signals von 5000, respektive 1500 AU. Zudem ist in beiden Proben das Hintergrundrauschen durch Signale anderer Peptide deutlich ausgeprägter und das saure Peptid wird in beiden Proben klar detektiert.

Dies zeigt, dass es im Gegensatz zu den nach dem Stand der Technik durchgeführten Verfahren mit dem erfindungsgemäßen Verfahren erstmals möglich ist, auch saure Peptide quantitativ abzutrennen und außerdem das Phosphopeptid mit sehr hoher Signalintensität nachzuweisen:

## Patentansprüche

1. Verfahren zur Anreicherung von Phosphopeptiden, **gekennzeichnet durch** folgende Verfahrensschritte
a) Bereitstellung eines Trägermaterials mit Chelatliganden auf Basis von Silica
b) Aktivierung des Trägermaterials aus Schritt a) mit Übergangsmetallionen, Oxiden oder Oxidhydraten von Übergangsmetallionen oder dreiwertigen Ionen von Metallen der dritten Hauptgruppe
c) Inkontaktbringen einer phosphopeptidhaltigen Probe mit dem aktivierten Trägermaterial in Anwesenheit eines Bindungspuffers
d) Entfernen des Überstands bestehend aus dem Bindungspuffer und dem nicht gebundenen Teil der Probe
e) optional Waschen des Trägermaterials
f) Elution der Phosphopeptide mit einem Elutionspuffer, der einen pH-Wert >10 aufweist und Alkali-, Erdalkali- oder Ammoniumsalze des Thiocyanats, der Säuren der Komplexliganden Nitrito, Isocyano, Nitril, Isocyanato, Isothiocyanato, Azido, Ethylendiamin, Isonitril, Fulminato und Cyano und/oder der Sauerstoffsäuren des Phosphors, Schwefels, Vanadiums, Rutheniums, Niobs, Tantals, Wolframs oder des Molybdäns enthält und/oder Chelatbildner wie EDTA, EGTA oder Salicylsäure.

2. Verfahren nach Anspruch 1, **dadurch gekennzeichnet, dass** die Aktivierung in Schritt b) mit Eisen (III)-Ionen oder Zirkonium (IV)-Ionen erfolgt.

3. Verfahren nach Anspruch 1 oder 2, **dadurch gekennzeichnet, dass** die Elution in Schritt f) mit einem Elutionspuffer erfolgt, der Alkali-, Erdalkali- oder Ammoniumsalze von Sauerstoffsäuren des Phosphors oder von Thiocyanat in einer Konzentration zwischen 0,005 und 2 mol/l enthält.

4. Verfahren nach einem oder mehreren der Ansprüche 1 bis 3, **dadurch gekennzeichnet, dass** die in Schritt f) eluierten Phosphopeptide direkt massenspektrometrisch, Dünnschicht-chromatographisch oder durch Sequenzanalyse untersucht werden.

5. Verfahren nach einem oder mehreren der Ansprüche 1 bis 4, **dadurch gekennzeichnet, dass** in Schritt a) ein Trägermaterial mit Chelatliganden gemäß Formel Ia und/oder Ib bereitgestellt wird 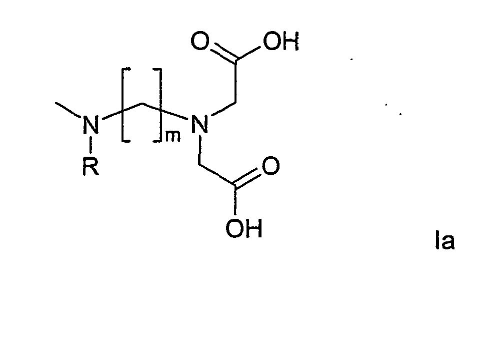 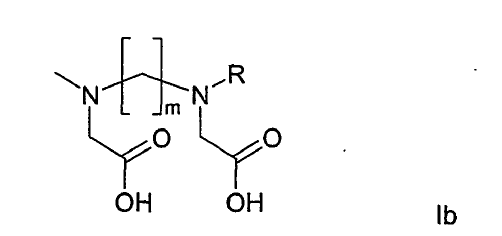 mit
R = C1 bis C6 Alkyl oder C5 bis C18 Aryl, optional ein oder mehrfach substitutiert z.B. mit Hydroxy-, C1-C4-Alkoxy-, Amino-, Alkylamino-, CN- oder Halogenresten,
m = 2 bis 8, wobei ein oder mehrere nicht benachbarte C-Atome durch O, NH, S oder -C=C- ersetzt sein können.

6. Verfahren nach einem oder mehreren der Ansprüche 1 bis 5, **dadurch gekennzeichnet, dass** in Schritt a) ein Trägermaterial mit Chelatligand bereitgestellt wird, das aus Magnetit-Partikeln besteht, deren Oberfläche zumindest teilweise mit Silica belegt ist.

7. Kit zur Anreicherung von Phosphopeptiden zumindest enthaltend ein Trägermaterial mit Chelatligand auf Silica-Basis und einen Elutionspuffer, der einen pH-Wert > 10 aufweist und Alkali-, Erdalkali- oder Ammoniumsalze des Thiocyanats, der Säuren der Komplexliganden Nitrito, Isocyano, Nitril, Isocyanato, Isothiocyanato, Azido, Ethylendiamin, Isonitril, Fulminato oder Cyano und/oder der Sauerstoffsäuren des Phosphors, Schwefels, Vanadiums, Rutheniums, Niobs, Tantals, Wolframs und/oder Molybdäns enthält und/oder Chelatbildner wie EDTA, EGTA oder Salicylsäure.

8. Kit nach Anspruch 7, **dadurch gekennzeichnet, dass** das Trägermaterial mit Chelatligand mit Eisen (III) oder Zirkonium (IV)-Ionen aktiviert ist.

9. Kit nach einem der Ansprüche 7 oder 8, **dadurch gekennzeichnet, dass** der Kit als Trägermaterial Magnetit-Partikel enthält, die zumindest teilweise mit Silica überzogen sind.

10. Kit nach einem oder mehreren der Ansprüche 7 bis 9, **dadurch gekennzeichnet, dass** der Kit ein Trägermaterial mit Chelatliganden entsprechend Formel Ia und/oder Ib, mit R = Methyl und m = 2, enthält.

11. Kit nach einem oder mehreren der Ansprüche 7 bis 10, **dadurch gekennzeichnet, dass** der Kit als Elutionspuffer einen Puffer enthält, der Alkali-, Erdalkali- oder Ammoniumsalze von Sauerstoffsäuren des Phosphors oder von Thiocyanat in einer Konzentration zwischen 0,005 und 2 mol/l enthält.

12. Trägermaterial, **dadurch gekennzeichnet, dass** es Chelatliganden der Formel Ia und/oder Ib enthält. 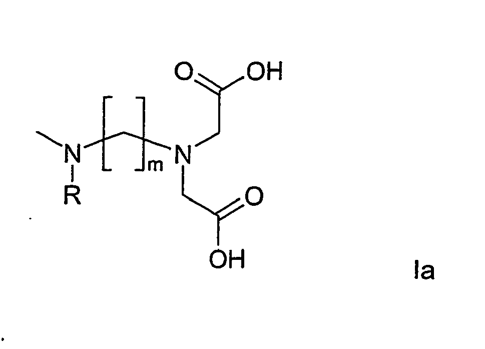 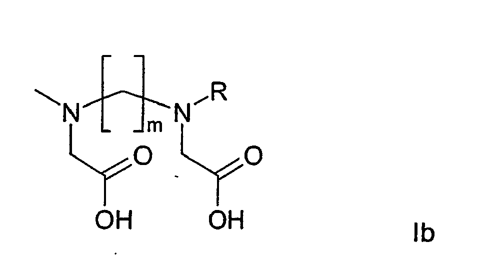 mit
R = C1 bis C6 Alkyl oder C5 bis C18 Aryl, optional ein oder mehrfach substitutiert z.B. mit Hydroxy-, C1-C4-Alkoxy-, Amino-, Alkylamino-, CN- oder Halogenresten,
m = 2 bis 8, wobei ein oder mehrere nicht benachbarte C-Atome durch O, NH, S oder -C=C- ersetzt sein können.

13. Trägermaterial nach Anspruch 12, **dadurch gekennzeichnet, dass** R Ethyl oder Methyl ist.

14. Trägermaterial nach Anspruch 12 oder 13, **dadurch gekennzeichnet, dass** m = 2 ist.

## Claims

1. Method for the enrichment of phosphopeptides, **characterised by** the following method steps:
a) provision of a support material having chelate ligands based on silica
b) activation of the support material from step a) using transition-metal ions, oxides or oxide hydrates of transition-metal ions or trivalent ions of metals from the third main group
c) bringing a phosphopeptide-containing sample into contact with the activated support material in the presence of a binding buffer
d) removal of the supernatant consisting of the binding buffer and the unbound part of the sample
e) optionally washing of the support material
f) elution of the phosphopeptides with an elution buffer which has a pH > 10 and comprises alkali metal, alkaline earth metal or ammonium salts of thiocyanate, of acids of the complex ligands nitrito, isocyano, nitrile, isocyanato, isothiocyanato, azido, ethylenediamine, isonitrile, fulminato and cyano and/or of the oxygen acids of phosphorus, sulfur, vanadium, ruthenium, niobium, tantalum, tungsten or of molybdenum, and/or chelating agents, such as EDTA, EGTA or salicylic acid.

2. Method according to Claim 1, **characterised in that** the activation in step b) is carried out using iron(III) ions or zirconium(IV) ions.

3. Method according to Claim 1 or 2, **characterised in that** the elution in step f) is carried out with an elution buffer which comprises alkali metal, alkaline earth metal or ammonium salts of oxygen acids of phosphorus or of thiocyanate in a concentration of between 0.005 and 2 mol/l.

4. Method according to one or more of Claims 1 to 3, **characterised in that** the phosphopeptides eluted in step f) are investigated directly by mass spectrometry, thin-layer chromatography or by sequence analysis.

5. Method according to one or more of Claims 1 to 4, **characterised in that** a support material having chelate ligands of the formula Ia and/or Ib is provided in step a) 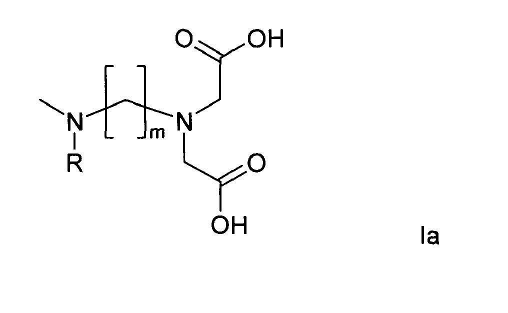 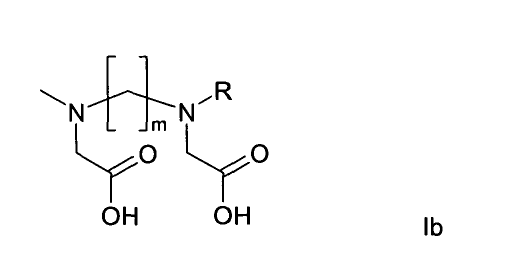 where
R = C1 to C6 alkyl or C5 to C18 aryl, optionally mono- or polysubstituted, for example by hydroxyl, C1-C4-alkoxy, amino, alkylamino, CN or halogen radicals,
m = 2 to 8, where one or more non-adjacent C atoms may be replaced by O, NH, S or -C=C-.

6. Method according to one or more of Claims 1 to 5, **characterised in that** a support material having a chelate ligand which consists of magnetite particles whose surface is at least partly covered by silica is provided in step a).

7. Kit for the enrichment of phosphopeptides, at least containing a support material having a chelate ligand based on silica and an elution buffer which has a pH > 10 and comprises alkali metal, alkaline earth metal or ammonium salts of thiocyanate, of acids of the complex ligands nitrito, isocyano, nitrile, isocyanato, isothiocyanato, azido, ethylenediamine, isonitrile, fulminato or cyano and/or of the oxygen acids of phosphorus, sulfur, vanadium, ruthenium, niobium, tantalum, tungsten and/or molybdenum, and/or chelating agents, such as EDTA, EGTA or salicylic acid.

8. Kit according to Claim 7, **characterised in that** the support material having a chelate ligand has been activated using iron(III) or zirconium(IV) ions.

9. Kit according to one of Claims 7 or 8, **characterised in that** the kit contains, as support material, magnetite particles which are at least partly coated with silica.

10. Kit according to one or more of Claims 7 to 9, **characterised in that** the kit contains a support material having chelate ligands conforming to the formula Ia and/or Ib, where R = methyl and m = 2.

11. Kit according to one or more of Claims 7 to 10, **characterised in that** the kit contains, as elution buffer, a buffer which comprises alkali metal, alkaline earth metal or ammonium salts of oxygen acids of phosphorus or of thiocyanate in a concentration of between 0.005 and 2 mol/l.

12. Support material, **characterised in that** it contains chelate ligands of the formula Ia and/or Ib 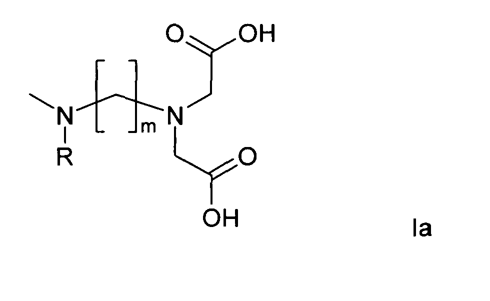 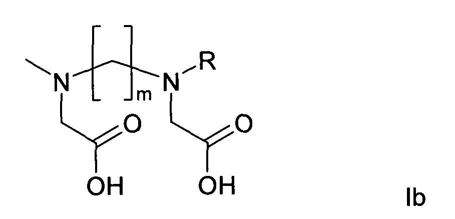 where
R = C1 to C6 alkyl or C5 to C18 aryl, optionally mono- or polysubstituted, for example by hydroxyl, C1-C4-alkoxy, amino, alkylamino, CN or halogen radicals,
m = 2 to 8, where one or more non-adjacent C atoms may be replaced by O, NH, S or -C=C-.

13. Support material according to Claim 12, **characterised in that** R is ethyl or methyl.

14. Support material according to Claim 12 or 13, **characterised in that** m = 2.

## Revendications

1. Méthode d'enrichissement en phosphopeptides, **caractérisée par** les étapes de méthode suivante :
a) la fourniture d'un matériau support ayant des ligands chélatés à base de silice
b) l'activation du matériau support issu de l'étape a) à l'aide d'ions de métaux de transition, d'oxydes ou d'hydrates d'oxydes d'ions de métaux de transition ou d'ions trivalents de métaux issus du troisième groupe principal
c) la mise en contact d'un échantillon contenant des phosphopeptides avec le matériau support activé en présence d'un tampon de fixation
d) l'élimination du surnageant constitué du tampon de fixation et de la partie non fixée de l'échantillon
e) éventuellement le lavage du matériau support
f) l'élution des phosphopeptides par un tampon d'élution ayant un pH > 10 et comprenant des sels de métaux alcalins, de métaux alcalino-terreux ou d'ammonium de thiocyanate, d'acides des ligands complexes nitrito, isocyano, nitrile, isocyanato, isothiocyanato, azido, éthylènediamine, isonitrile, fulminato et cyano et/ou d'acides oxygénés de phosphore, soufre, vanadium, ruthénium, niobium, tantale, tungstène ou molybdène, et/ou des agents chélateurs, tels que l'EDTA, l'EGTA ou l'acide salicylique.

2. Méthode selon la revendication 1, **caractérisée en ce que** l'activation dans l'étape b) est effectuée à l'aide d'ions de fer(III) ou d'ions de zirconium(IV).

3. Méthode selon la revendication 1 ou 2, **caractérisée en ce que** l'élution dans l'étape f) est effectuée par un tampon d'élution comprenant des sels de métaux alcalins, de métaux alcalino-terreux ou d'ammonium d'acides oxygénés de phosphore ou de thiocyanate en une concentration comprise entre 0,005 et 2 mol/l.

4. Méthode selon l'une ou plusieurs des revendications 1 à 3, **caractérisée en ce que** les phosphopeptides élués dans l'étape f) sont étudiés directement par spectrométrie de masse, chromatographie sur couche mince ou par analyse de séquence.

5. Méthode selon l'une ou plusieurs des revendications 1 à 4, **caractérisée en ce qu'**un matériau support ayant des ligands chélatés de formule Ia et/ou Ib est fourni dans l'étape a) 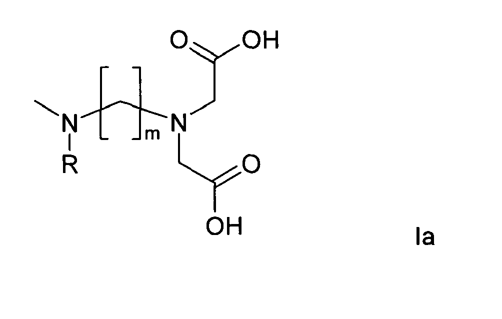 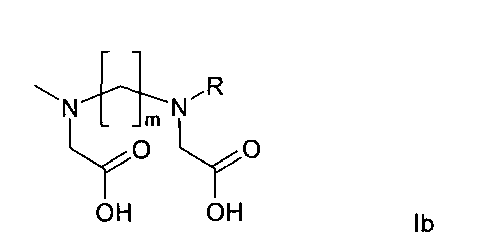 où
R = C1-C6-alkyle ou C5-C18-aryle, éventuellement mono- ou polysubstitué, par exemple par des radicaux hydroxyle, C1-C4-alcoxy, amino, alkylamino, CN ou halogène,
m = 2 à 8, où un ou plusieurs atomes de C non adjacents peuvent être remplacés par O, NH, S ou -C=C-.

6. Méthode selon l'une ou plusieurs des revendications 1 à 5, **caractérisée en ce qu'**un matériau support ayant un ligand chélaté qui est constitué de particules de magnétite dont la surface est au moins partiellement recouverte de silice est fourni dans l'étape a).

7. Kit d'enrichissement en phosphopeptides, au moins contenant un matériau support ayant un ligand chélaté à base de silice et un tampon d'élution ayant un pH > 10 et comprenant des sels de métaux alcalins, de métaux alcalino-terreux ou d'ammonium de thiocyanate, d'acides des ligands complexes nitrito, isocyano, nitrile, isocyanato, isothiocyanato, azido, éthylènediamine, isonitrile, fulminato ou cyano et/ou d'acides oxygénés de phosphore, soufre, vanadium, ruthénium, niobium, tantale, tungstène et/ou molybdène, et/ou des agents chélateurs, tels que l'EDTA, l'EGTA ou l'acide salicylique.

8. Kit selon la revendication 7, **caractérisé en ce que** le matériau support ayant un ligand chélaté a été activé à l'aide d'ions de fer(III) ou de zirconium(IV).

9. Kit selon l'une des revendications 7 ou 8, **caractérisé en ce que** le kit contient, comme matériau support, des particules de magnétite qui sont au moins partiellement recouvertes de silice.

10. Kit selon l'une ou plusieurs des revendications 7 à 9, **caractérisé en ce que** le kit contient un matériau support ayant des ligands chélatés conformes à la formule la et/ou Ib, où R = méthyle et m = 2.

11. Kit selon l'une ou plusieurs des revendications 7 à 10, **caractérisé en ce que** le kit contient, comme tampon d'élution, un tampon comprenant des sels de métaux alcalins, de métaux alcalino-terreux ou d'ammonium d'acides oxygénés de phosphore ou de thiocyanate en une concentration comprise entre 0,005 et 2 mol/l.

12. Matériau support, **caractérisé en ce qu'**il contient des ligands chélatés de formule la et/ou Ib 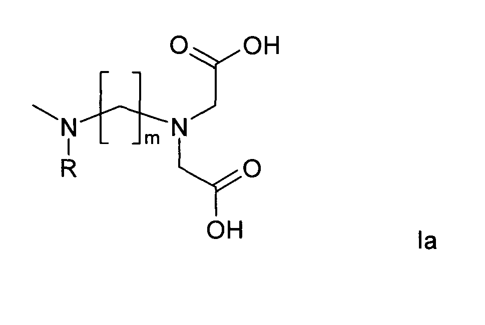 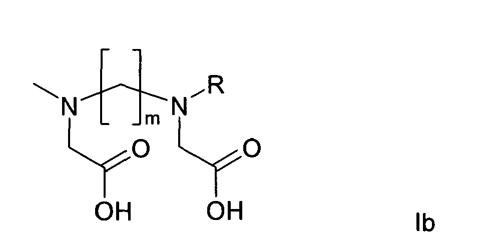 où
R = C1-C6-alkyle ou C5-C18-aryle, éventuellement mono- ou polysubstitué, par exemple par des radicaux hydroxyle, C1-C4-alcoxy, amino, alkylamino, CN ou halogène,
m = 2 à 8, où un ou plusieurs atomes de C non adjacents peuvent être remplacés par O, NH, S ou -C=C-.

13. Matériau support selon la revendication 12, **caractérisé en ce que** R est éthyle ou méthyle.

14. Matériau support selon la revendication 12 ou 13, **caractérisé en ce que** m = 2.
